Europäisches Patentamt

European Patent Office

Office européen des brevets

(11) Publication number: **0 182 448**
A2

# EUROPEAN PATENT APPLICATION

(21) Application number: 85202121.1

(22) Date of filing: 12.10.84

(51) Int. Cl.⁴: **C 12 N 15/00**, C 12 P 21/02,
C 12 Q 1/68, A 61 K 37/02,
C 07 K 13/00

(30) Priority: 24.08.84 US 644036

(43) Date of publication of application: 28.05.86
**Bulletin 86/22**

(84) Designated Contracting States: **AT BE CH DE FR GB LI
LU NL SE**

(60) Publication number of the earlier application in
accordance with Art. 76 EPC: **0162067**

(71) Applicant: **Genetics Institute, 225 Longwood Avenue,
Boston Massachusetts 02115 (US)**

(72) Inventor: **Toole, John J., Jr., 27 Lakeville Road, Jamaica
Plain Massachusetts 02130 (US)**

(74) Representative: **MacGregor, Gordon et al, ERIC POTTER
& CLARKSON 14 Oxford Street, Nottingham, NG1 5BP
(GB)**

(54) **Production of factor VIII and related products.**

(57) Preparations of recombinant DNA which code for cellular
production of human and porcine factor VIII:C and methods of
obtaining such DNA and expression thereof in bacteria and
eucaryotic cells is disclosed. New compounds, including deoxy-
ribonucleotides and ribonucleotides which are utilized in obtain-
ing clones which express factor VIII:C are also disclosed.

EP 0 182 448 A2

ACTORUM AG

PRODUCTION OF FACTOR VIII AND RELATED PRODUCTS

Prior Application

This application is a continuation-in-part application of U.S. Application Serial No. 546,650 filed on October 28, 1983.

Background of the Invention

This invention relates to the preparation of recombinant deoxyribonucleic acid (DNA) which codes for cellular production of human factor VIII:C, and of DNA which codes for porcine factor VIII:C, to methods of obtaining DNA molecules which code for factor VIII:C, and to expression of human and porcine factor VIII:C utilizing such DNA, as well as to novel compounds, including deoxyribonucleotides and ribonucleotides utilized in obtaining such clones and in achieving expression of human factor VIII:C. This invention also relates to human AHF and its production by recombinant DNA techniques.

Factor VIII:C is a blood plasma protein that is defective or absent in Hemophilia A disease. This disease is a hereditary bleeding disorder affecting approximately one in 20,000 males. Factor VIII:C has also been known or referred to as factor VIII, the antihemophilic factor (AHF), antihemophilic globulin (AHG), hemophilic factor A, platelet cofactor, thromboplastinogen, and thrombocytolysin. It is referred to as "Factor VIII:C", to indicate that it is the compound which affects clotting activity. As used herein, "factor VIII:C" and "AHF" are synonymous.

Although the isolation of AHF from blood plasma has been described in the literature, the precise structure of AHF has not previously been identified, due in part to the unavailability of sufficient quantities of pure material, and the proteolytic nature of many contaminants and purification agents. While some quantities of impure AHF have been available as a concentrated preparation processed from fresh-frozen human plasma, the extremely low concentration of AHF in human plasma and the high cost of obtaining and processing human plasma make the cost of this material prohibitive for any extensive treatment of hemophilia.

The present invention makes it possible to produce human AHF using recombinant DNA techniques.

AHF, like other proteins, is comprised of some twenty different amino acids arranged in a specific array. By using gene manipulation techniques, a method has been developed which enables production of AHF by identifying and cloning the gene which codes for the human AHF protein, cloning that gene, incorporating that gene into a recombinant DNA vector, transforming a suitable host with the vector which includes that gene, expressing the human AHF gene in such host, and recovering the human AHF produced thereby. Similarly, the present invention makes it possible to produce porcine AHF by recombinant DNA techniques, as well as providing products and methods related to such porcine AHF production.

Recently developed techniques have made it possible to employ microorganisms, capable of rapid and abundant growth, for the synthesis of commercially useful proteins and peptides, regardless of their source in nature. These techniques make it possible to genetically endow a suitable microorganism with the ability to synthesize a protein or peptide normally made by another organism. The technique makes use of fundamental relationships which exist in all living organisms between the genetic material, usually DNA, and the proteins synthesized by the organism. This relationship is such that production of the amino acid sequence of the protein is coded for by a series of three nucleotide sequences of the DNA. There are one or more trinucleotide sequence groups (called codons) which specifically code for the production of each of the twenty amino acids most commonly occurring in proteins. The specific relationship between each given trinucleotide sequence and the corresponding amino acid for which it codes constitutes the genetic code. As a consequence, the amino acid sequence of every protein or peptide is reflected by a corresponding nucleotide sequence, according to a well understood relationship. Furthermore, this sequence of nucleotides can, in principle, be translated by any living organism. For a discussion of the genetic code, see J. D. Watson, Molecular Biology of the Gene, (W. A. Benjamin, Inc., 1977), the disclosure of which is incorporated herein by reference, particularly at 347-77; C. F. Norton, Microbiology (Addison, Wesley 1981), and U. S. Patent No. 4,363,877, the disclosure of which is incorporated herein by reference.

The twenty amino acids from which proteins are made, are phenylalanine (hereinafter sometimes referred to as "Phe" or "F"), leucine ("Leu", "L"), isoleucine ("Ile", "I"), methionine ("Met", "M"), valine ("Val", "V"), serine ("Ser", "S"), proline ("Pro", "P"), threonine ("Thr", "T"), alanine ("Ala", "A"), tyrosine ("Tyr", "Y"), histidine ("His", "H"), glutamine ("Gln", "Q"), asparagine ("Asp", "N"), glutamic acid ("Glu", "E"), cysteine ("Cys", "C"), tryptophane ("Trp", "W"), arginine ("Arg", "R") and glycine ("Gly", "G"). The amino acids coded for by the various combinations of trinucleotides which may be contained in a given codon may be seen in Table 1:

## TABLE 1

### The Genetic Code

| First Position | Second Position | | | | Third Position |
|:---:|:---:|:---:|:---:|:---:|:---:|
| | T | C | A | G | |
| T | Phe | Ser | Tyr | Cys | T |
| | Phe | Ser | Tyr | Cys | C |
| | Leu | Ser | Stop* | Stop* | A |
| | Leu | ·Ser | Stop* | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asp | Ser | T |
| | Ile | Thr | Asp | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

*The "Stop" or termination codon terminates the expression of the protein.

Knowing the deoxyribonucleotide sequence of the gene or DNA sequence which codes for a particular protein allows the exact description of that protein's amino acid sequence. However, the converse is not true; while methionine is coded for by only one codon, the other amino acids can be coded for by up to six codons (e.g. serine), as is apparent from Table 1. Thus there is considerable ambiguity in predicting the nucleotide sequence from the amino acid sequence.

In sum, prior to the present invention, very little was known about the structure of AHF, and, despite substantial work over many years, those skilled in this art were unable to determine the structure of AHF, or of its gene, or provide any procedure by which AHF could be produced in substantially pure form in substantial quantities.

The method described herein by which the gene for human AHF is cloned and expressed includes the following steps:

    (1)  Purification of porcine AHF;

    (2)  Determination of the amino acid sequence of porcine AHF;

    (3)  Formation of oligonucleotide probes, and use of those probes to identify and/or isolate at least a fragment of the gene which codes for porcine AHF;

    (4)  Use of the porcine AHF gene fragment to identify and isolate human genetic material which codes for human AHF;

    (5)  Using the previously described AHF DNA fragments to determine the site of synthesis of AHF from among the various mammalian tissues;

    (6)  Producing cDNA segments which code for human and porcine AHF, using messenger RNA obtained from the tissue identification in step 5;

0182448

(7)  Constructing full length human and porcine cDNA clones from the cDNA segments produced in step 6, e.g. by ligating together cDNA segments which were cut by the same restriction enzymes;

(8)  Forming DNA expression vectors which are capable of directing the synthesis of AHF;

(9)  Transforming a suitable host with the expression vectors bearing the full length cDNA for human or porcine AHF;

(10) Expressing human or porcine AHF in the host; and

(11) Recovering the expressed AHF.

In the course of this work, a new technique of screening a genomic DNA library has been developed utilizing oligonucleotide probes based on the amino acid sequences contained in the AHF molecule.

The invention includes the above methods, along with the various nucleotides, vectors, and other products made in connection therewith.

## Brief Summary of The Drawings

Figure 1 is a depiction of the amino acid sequence (A) determined for the amino terminal sequence of the 69,000 dalton thrombin cleavage product described in Example 1.  The first residue was not identifiable.  This sequence is compared with sequence (B) deduced from the nucleotide sequence of the porcine AHF exon described in Example 3, and with the human AHF exon sequence (C) described in Example 4.

Figure 2 illustrates the amino acid sequence, shown in single letter code, of bovine thrombin digestion fragments of (A) the amino terminus and (B) a 40 Kd thrombin cleavage product of the 166 Kd porcine AHF fragment isolated by Fass et al., _infra_.

Figure 3 illustrates the design of an oligonucleotide probe for the identification and isolation of at least a portion of the porcine gene which codes for AHF.

Figure 4 illustrates the DNA sequence for a Sma I DNA fragment (34-S1) which contains a porcine AHF exon, derived from bacteriophage PB34 described in Example 3.

Figure 5 is a representation of the DNA sequence of the Hae III insert 34-H1 bearing the exon for porcine AHF, as described in Example 3. This sequence is included within the longer sequence shown in Figure 4 and corresponds to nucleotides 250-615 of the sequence in Figure 4.

Figure 6 is a representation of the DNA sequence along with the deduced amino acid sequence for nucleotides 34-84 for a portion of the Sau 3AI insert of clone 25-S1, showing a portion of the exon for human AHF, as described in Example 4.

Figure 7 illustrates the DNA nucleotide sequence (shown in one strand only) which contains the entire sequence coding for human AHF, along with the deduced amino acid sequence for human AHF.

## Detailed Description of the invention

The following definitions are supplied in order to facilitate the understanding of this case. To the extent that the definitions vary from meanings circulating within the art, the definitions below are to control.

Amplification means the process by which cells produce gene repeats within their chromosomal DNA.

Cotransformation means the process of transforming a cell with more than one exogenous gene foreign to the cell, one of which confers a selectable phenotype on the cell.

Downstream means the direction going towards the 3' end of a nucleotide sequence.

An enhancer is a nucleotide sequence that can potentiate the transcription of genes independent of the identity of the gene, the position of the sequence in relation to the gene, or

0182448

the orientation of the sequence.

A gene is a deoxyribonucleotide sequence coding for a given mature protein. For the purposes herein, a gene shall not include untranslated flanking regions such as RNA transcription initiation signals, polyadenylation addition sites, promoters or enhancers.

A selection gene is a gene that confers a phenotype on cells which express the gene as a detectable protein.

A selection agent is a condition or substance that enables one to detect the expression of a selection gene.

Phenotype means the observable properties of a cell as expressed by the cellular genotype.

Genotype means the genetic information contained within a cell as opposed to its expression, which is observed as the phenotype.

Ligation is the process of forming a phosphodiester bond between the 5' and 3' ends of two DNA strands. This may be accomplished by several well known enzymatic techniques, including blunt end ligation by T4 ligase.

Orientation refers to the order of nucleotides in a DNA sequence. An inverted orientation of a DNA sequence is one in which the 5' to 3' order of the sequence in relation to another sequence is reversed when compared to a point of reference in the DNA from which the sequence was obtained. Such points of reference can include the direction of transcription of other specified DNA sequences in the source DNA or the origin of replication of replicable vectors containing the sequence.

Transcription means the synthesis of RNA from a DNA template.

Transformation means changing a cell's genotype by the cellular uptake of exogenous DNA. Transformation may be detected in some cases by an alteration in cell phenotype. Transformed cells are called transformants. Pre-transformation cells are referred to as parental cells.

Translation means the synthesis of a polypeptide from messenger RNA (mRNA).

The present invention permits, for the first time, the identification and isolation of porcine Factor VIII:C gene by recombinant DNA techniques. It also permits, for the first time, the isolation and identification of the gene which encodes human factor VIII:C by recombinant DNA techniques, by taking advantage of the homology between porcine AHF DNA and human AHF DNA in order to locate and isolate the human gene for AHF. This route to cDNA clones for producing AHF via homology with the porcine AHF gene avoids the tedious time consuming and expensive need to purify human AHF, which is highly expensive and essentially unavailable.

Porcine AHF is first highly purified, most preferably by a monoclonal antibody purification technique such as that disclosed by David Fass et al, "Monoclonal Antibodies to Porcine AHF Coagulant and Their Use in the Isolation of Active Coagulent Protein", Blood 59: 594-600 (1982), and Knutsen et al., "Porcine Factor VIII:C prepared by affinity Interaction with Von Willebrand Factor and Heterologous Antibodies," Blood, 59: 615-24 (1982), the disclosures of both of which are incorporated herein by reference. Porcine factor VIII:C polypeptides are bound to anti-VIII:C monoclonal antibodies which are immobilized on a suitable affinity chromatography column. Two large molecular weight polypeptides, having molecular sizes of about 166 and 130 Kd, are eluted with ethylenediamine tetraacetic acid. Another protein segment having a molecular weight of about 76 Kd, is then eluted from the column, utilizing about 50% ethylene glycol. The partial amino acid sequences of these polypeptides, and/or of fragments of these polypeptides obtained in a known manner, e.g. from enzymatic digestion of the proteins, using bovine thrombin or other suitable agents to break up the proteins, are then determined by known methods of analysis. Based on the amino acid sequence of these materials, oligonucleotide probes are synthesized, at least some of which will hybridize with DNA segments which code for the corresponding segment of AHF. These oligonucleotides are then used to screen for segments of the gene which code for porcine AHF.

Once a portion of the porcine gene for AHF is obtained, that recombinant material is used to screen a human genomic DNA library to locate and isolate the gene which codes for human AHF. In this procedure, it is established that there are substantial similarities between human AHF and porcine AHF, and advantage is taken of those similarities to isolate and identify the human factor VIII:C gene or gene segments.

The similarities in the porcine and human proteins are attributable to corresponding similarities in the DNA sequences which code for the amino acid sequences. The genetic materials coding for the AHF proteins in humans and pigs are identical at a high percentage of positions, and thus exhibit hybridization when subjected to the procedure of, for example, Benton and Davis, "Screening gt Recombinant Clones by Hybridization to Single Plaques In Situ," Science, 196:180 (1977), the disclosure of which is incorporated herein by reference.

The cloned segments of the gene for human AHF are then used to identify a source for AHF mRNA from among various human tissues. Similarly, one or more of the cloned segments for porcine AHF are used to screen potential tissue sources for porcine mRNA. This step is accomplished by conventional procedures involving RNA extraction, gel electrophoresis, transfer to nitrocellulose sheets, and hybridization to radiolabelled cloned DNA as described for example in Maniatis, et. al., Molecular Cloning, A Laboratory Manual, (Cold Spring Harbor Laboratory, 1982). Once this tissue source is identified, cDNA libraries are prepared (in either plasmid or preferably bacteriophage lambda vectors, both of which are generally available) and screened for AHF cDNA clones as described below. The process of cDNA library screening is repeated until a set of

0182448

cDNA clones is obtained which, by DNA sequence determintion, is shown to comprise the entire DNA gene sequence encoding AHF.

Once the full length human or porcine AHF cDNA clone is obtained, known and appropriate means are utilized to express the AHF protein, e.g. insertion into an appropriate vector, and transfection into an appropriate host, selection of transformed cells (transformants), and culture these transformants, to express AHF activity.

Host-vector systems for the expression of AHF may be procaryotic, but the complexity of AHF makes the preferred expression system eucaryotic, preferably (at least for biologically-active AHF having clotting activity) a mammalian one. This is easily accomplished by eucaryotic (usually mammalian or vertebrate cells) transformation with a suitable AHF vector. Eucaryotic transformation is in general a well-known process, and may be accomplished by a variety of standard methods. These include the use of protoplast fusion, DNA microinjection, chromosome transfection, lytic and nonlytic viral vectors (For example, Mulligan et al., "Nature" (London) 277:108-114 (1979), cell-cell fusion (Fournier et al., "Proc. Nat. Acad. Sci." 74:319-323 (1977), lipid structures (U.S. patent 4,394,448) and cellular endocytosis of DNA precipitates (Bachetti et al., "Proc. Nat. Acad. Sci. 74:1590-1594 (1977). Other eucaryotic cells, such as yeasts or insect cells, may also be used to advantage.

Transformation which is mediated by lytic viral vectors is efficient but is disadvantageous for a number of reasons: The maximum size of transfected DNA is limited by the geometry of viral capsid packing, the exogenous genes are frequently deleted during viral replication, there is a requirement for helper virus or specialized hosts, host cells must be permissive, and the hosts are killed in the course of the viral infection.

Nonlytic transformations are based on the transcription and translation of virus vectors which have been incorporated into a cell line as a stable episome. These systems generally require unique cell lines and suffer from a number of disadvantages. See "Trends in Biochemical Sciences", June 1983, pp. 209-212.

On the other hand, other transformation methods in which extrachromosomal DNA is taken up into the chromosomes of host cells have been characterized by low frequencies of transformation and poor expression levels. These initial difficulties were ameliorated by transformation with genes which inheritably confer selectable phenotypes on the small subpopulation of cells that are in fact transformed (selection genes). The entire population of transformed cells can be grown under conditions favoring cells having acquired the phenotype, thus making it possible to locate transformed cells conveniently. Thereafter, transformants can be screened for the capability to more intensely express the phenotype. This is accomplished by changing a selection agent in such a way as to detect higher expression.

Selection genes fall into three categories: Detectably amplified selection genes, dominant selection genes, and detectably amplified dominant selection genes.

Detectably amplified selection genes are those in which amplification can be detected by exposing host cells to changes in the selection agent. Detectably amplified genes which are not dominant acting generally require a parental cell line which is genotypically deficient in the selection gene. Examples include the genes for hydroxymetholglutanyl CoA reductase (Sinensky, "Biochem. Biophys. Res. Commun" 78:863 (1977), ribonucleotide reductase (Meuth et al. "Cell:3:367 (1943), aspartate transcarbamylase; (Kemp et al. "Cell" 9:541 (1976), adenylate deaminase (DeBatisse et al. "Mol and Cell Biol." 2(11):1346-1353 (1982) mouse dihydrofolate reductase (DHFR) and, with a defective promoter, mouse thymidine kinase (TK).

Dominant selection genes are those which are expressed in transformants regardless of the genotype of the parental cell. Most dominant selection genes are not detectably amplified because the phenotype is so highly effective in dealing with the selection agent that it is difficult to discriminate among cell lines that have or have not amplified the gene. Examples of dominant selection genes of this type include the genes for procaryotic enzymes such as xanthine-guanine phosphoribosyltransferase (Mulligan et al. "Proc. Nat. Acad. Sci." 78[4]:2072-2076 (1981) and aminoglycoside 3' - phosphotransferase (Colbere-Garapin et al., "J. Mol. Biol.", 150:1-14 (1981).

Some dominant selection genes also are detectably amplified. Suitable examples include the mutant DHFR gene described by Haber et al., "Somatic Cell Genet." 4:499-508 (1982), cell surface markers such as HLA antigens and genes coding for enzymes such as specific esterases that produce fluorescent or colored products from fluorogenic or chromogenic substrates as is known in the art.

Detectably-amplified, dominant selection genes are preferred for use herein. It should be understood that a dominant selection gene in some cases can be converted to a detectably amplified gene by suitable mutations in the gene.

Selection genes at first were of limited commercial utility. While they enabled one to select transformants having the propensity to amplify uptaken DNA, most selection genes produced products of no commercial value. On the other hand, genes for products which were commercially valuable generally did not confer readily selectable (or even detectable) phenotypes on their transformants. This would be the case, for example, with enzymes or hormones which do not provide transformed cells with unique nutrient metabolic or detoxification capabilities. Most proteins of commercial interest fall into this group, e.g. hormones, proteins participating in blood coagulation and fibrinolytic enzymes.

0182448

Subsequently it was found that eucaryotic cells having the propensity to be transformed with and amplify the selection gene would do the same in the case of the product gene. By following the selection gene one could identify a subpopulation of transformant cells which coexpress and coamplify the product gene along with the selection gene. It has been the practice to culture the transformants in the presence of the selection agent and to conclude that transformants having increased expression of the selection gene will also show increased expression of the product gene. This is not always the case, as will be more fully explored below. Axel et al. (U.S. patent 4,399,216) use the term cotransformation to describe the process of transforming a cell with more than one different gene, whether by vector systems containing covalently linked or unlinked genes, and in the latter case whether the genes are introduced into host cells sequentially or simultaneously. Cotransformation should "allow the introduction and stable integration of virtually any defined gene into cultured cells" (Wigler et al. "Cell", 16:777-785, (1975), and "by use of the cotransformation process it is possible to produce eucaryotic cells which synthesize desired proteinaceous and other materials" (U.S. patent 4,399,216, column 3, lines 37-42).

Transformation Vectors

Vectors used in AHF cotransformation will contain a selection gene and the AHF gene. In addition there usually will be present in the transformation or cotransformation vectors other elements such as enhancers, promoters, introns, accessory DNA, polyadenylation sites and 3' noncoding regions as will be described below.

Suitable selection genes are described above. It is preferred that the selection agent be one that prevents cell growth in the absence of the selection gene. That way, revertant cells in large scale culture that lose the selection gene (and presumably the AHF gene as well) will not over-grow the fermentation. However, it would be desirable in the commercial production of AHF to avoid the use of cell toxins, thereby simplifying the product purification steps. Thus, a desirable selection gene would be one that enables transformants to use a nutrient critical for growth that they otherwise would not be able to use. The TK gene described above is an example.

Two classes of vectors have been employed in cotransformation. The first class are the unlinked vectors. Here the selection gene and the AHF gene are not covalently bound. This vector class is preferred because the step of ligating or otherwise bonding the two genes is not required. This simplifies the transformation process because the selection and product genes usually are obtained from separate sources and are not ligated in their wild-type environment. In addition, the molar ratio of the AHF and selection genes employed during cotransformation can be adjusted to increase cotransformation efficiency.

The second class of cotransformation vectors are linked vectors. These vectors are distinguished from unlinked vectors in that the selection and AHF genes are covalently bound, preferably by ligation.

The vectors herein may also include enhancers. Enhancers are functionally distinct from promoters, but appear to operate in concert with promoters. Their function on the cellular level is not well understood, but their unique characteristic is the ability to activate or potentiate transcription without being position or orientation dependent. Promoters need to be upstream of the gene, while enhancers may be present upstream or 5' from the promoter, within the gene as an intron, or downstream from the gene between the gene and a polyadenylation site or 3' from the polyadenylation site. Inverted promoters are not functional, but inverted enhancers are. Enhancers are cis-acting, i.e., they have an effect on promoters only if they are present on the same DNA strand. For a general discussion of enhancers see Khoury et al., "Cell" 33:313-314 (1983).

Preferred enhancers are obtained from animal viruses such as simian virus 40, polyoma virus, bovine papilloma virus, retrovirus or adenovirus. Viral enhancers may be obtained readily from publically available viruses. The enhancer regions for several viruses, e.g., Rous sarcoma virus and simian virus 40, are well known. See Luciw et al., "Cell" 33:705-716 (1983). It would be a matter of routine chemistry to excise these regions on the basis of published restriction maps for the virus in question and, if necessary, modify the sites to enable splicing the enhancer into the vector as desired. For example, see Kaufman et al, "J. Mol. Biol.", 159:601-621 (1982) and "Mol. Cell Biol." 2(11):1304-1319 (1982) the disclosures of both of which are incorporated herein by reference. Alternatively, the enhancer may be synthesized from sequence data; the sizes of viral enhancers (generally less than about 150 bp) are sufficiently small that this could be accomplished practically.

Another element which should be present in the vector assembly is a polyadenylation splicing (or addition) site. This is a DNA sequence located downstream from the translated regions of a gene, shortly downstream from which in turn transcription stops and adenine ribonucleotides are added to form a polyadenine nucleotide tail at the 3' end of the messenger RNA. Polyadenylation is important in stabilizing the messenger RNA against degradation in the cell, an event that reduces the level of messenger RNA and hence the level of product protein.

Eucaryotic polyadenylation sites are well known. A concensus sequence exists among eucaryotic genes: The hexanucleotide 5'-AAUAAA-3' is found 11-30 nucleotides from the point at which polyadenylation starts. DNA sequences containing polyadenylation sites may be obtained from viruses in accord with published reports. Exemplary polyadenylation sequences can be obtained from mouse beta-globulin, and simian virus 40 late or early region genes, but viral polyadenylation sites are preferred. Since these sequences are known, they may be synthesized _in vitro_ and ligated to the vectors in conventional fashion.

A polyadenylation region must be located downstream from either the AHF and/or selection gene, but may be ligated to either gene. It may be ligated to the selection gene only, and not the product gene, and this will be the case whether the vectors are linked or unlinked. The sequence which separates the polyadenylation site from the translational stop codon is preferably an untranslated DNA oligonucleootide such as an unpromoted eucaryotic gene. Since such oligonucleotides and genes are not endowed with a promoter they will not be expressed. The oligonucleotide should extend for a considerable distance, on the order of up to about 1,000 bases, from the stop codon to the polyadenylation site. This 3' untranslated oligonucleotide generally results in an increase in product yields. The vector may terminate from about 10 to about 30 bp downstream from the concensus sequence, but it is preferable to retain the 3' sequences found downstream from the polyadenylation site in its wild-type environment. These sequences typically extend about from 200 to 600 base pairs downstream from the polyadenylation site.

The vectors described herein may be synthesized by techniques well known to those skilled in this art. The components of the vectors such selection genes, enhancers, promoters, and the like may be obtained from natural sources or synthesized as described above. Basically, if the components are found in DNA available in large quantity, e.g. components such as viral functions, or if they may be synthesized, e.g. polyadenylation sites, then with appropriate use of restriction enzymes large quantities of vector may be obtained by simply culturing the source organism, digesting its DNA with an appropriate endonuclease, separating the DNA fragments, identifying the DNA containing the element of interest and recovering same. Ordinarily, a transformation vector will be assembled in small quantity and then ligated to a suitable autonomously replicating synthesis vector such as a procaryotic plasmid or phage. The pBR322 plasmid may be used in most cases. See Kaufman et al., op. cit.

The synthesis vectors are used to clone the ligated transformation vectors in conventional fashion, e.g. by transfection of a permissive procaryotic organsim, replication of the synthesis vector to high copy number and recovery of the synthesis vector by cell lysis and separation of the synthesis vector from cell debris.

The resulting harvest of synthesis vector may be directly transfected into eucaryotic cells, or the transformation vector may be rescued from the synthesis vector by appropriate endonuclease digestion, separation by molecular weight and recovery of the transformation vector. Transformation vector rescue is not necessary so long as the remainder of the synthesis vector does not adversely affect eucaryotic gene amplification, transcription or translation. For example, the preferred synthesis vector herein is a mutant of the E. coli plasmid pBR322 in which sequences have been deleted that are deleterious to eucaryotic cells. See Kaufman et al., op. cit. Use of this mutant obviates any need to delete the plasmid residue prior to cotransformation.

Cotransformation, Selection and Detection of Amplification

The cells to be transformed may be any eucaryotic cell, including yeast protoplasts, but ordinarily a nonfungal cell. Primary explants (including relatively undifferentiated cells such as stem cells), and immortal and/or transformed cell lines are suitable. Candidate cells need not be genotypically deficient in the selection gene so long as the selection gene is dominant acting.

The cells preferably will be stable mammalian cell lines

0182448

as is discussed above. Cell lines that are known to stably integrate selection genes into their chromosomal DNA are best, for example Chinese hamster ovary (CHO) cell lines. Also useable are HeLa, COS monkey cells, melanoma cell lines such as the Bowes cell line, mouse L cells, mouse fibroblasts and mouse NIH 3T3 cells.

Cotransformation with unlinked vectors may be accomplished serially or simultaneously, (see U.S. patent 4,399,216). Methods for facilitating cellular uptake of DNA are described above. Microinjection of the vector into the cell nucleus will yield the highest transformation efficiencies, but exposing parental cells to DNA in the form of a calcium phosphate precipitate is most convenient. Considerably better cotransformation efficiencies result from cotransformation with a molar excess of product to selection gene, on the order of 100:1.

The population of cells that has been exposed to transforming conditions is then processed to identify the transformants. Only a small subpopulation of any culture which has been treated for cotransformation will exhibit the phenotype of the selection gene. The cells in the culture are screened for the phenotype. This can be accomplished by assaying the cells individually with a cell sorting device where the phenotype is one that will produce a signal, e.g. fluorescence upon cleavage of a fluorogenic substrate by an enzyme produced by the selection gene. Preferably, however, the phenotype enables only transformants to grow or survive in specialized growth media as is further discussed above.

Selection transformants then will be screened for ligation of the product gene into their chromosomes or for expression of the product itself. The former can be accomplished using Southern blot analysis, the latter by standard immunological or enzymatic assays.

Once the tranformants have been identified, steps are taken to amplify expression of the product gene by further cloning in the presence of a selection agent such as MTX.  See
U.S. Patent 4,399,216.

Cotransformants which may be produced in accordance with
the processes described herein are suitable for in vivo
transfections of higher organisms in accordance with known
techniques.  Primary explants or stable cell lines from a
potential host animal are cotransformed and inoculated into the
host or a substantially otherwise syngeneic host which is
genotypically deficient in the product protein.

The invention will be further understood with reference
to the following illustrative embodiments, which are purely
exemplary, and should not be taken as limitive of the true scope
of the present inevntion, as described in the claims.

Unless otherwise noted, restriction endonucleases are
utilized under the conditions and in the manner recommended by
their commercial suppliers.  Ligation reactions are carried on as
described by Maniatis et al., Molecular Cloning, A Laboratory
Manual, (Cold Spring Harbor Laboratory 1982) at 245-6, the
disclosure of which is incorporated herein by reference, using
the buffer described at page 246 thereof and using a DNA
concentration of 1-100 µg/ml, at a temperature of 23°C for blunt
ended DNA and 16°C for "sticky ended" DNA.  "Phosphatasing" as
described herein, refers to dephosphorylation of DNA, and is
carried out in the manner described by Maniatis et al., supra,
e.g. at page 133 et seq.  "Kinasing" refers to phosphorylation of
DNA.  Electrophoresis is done in 0.5-1.5% Agarose gels containing
90 mM Tris-borate, 10 mM EDTA.  "Nick-translating" refers to the
method for labeling DNA with $^{32}P$ as described by Rigby et al., J.
Mol. Biol., 113:237 (1977).  All radiolabeled DNA is labeled with
$^{32}P$, whatever labeling technique was used.

By "rapid prep" is meant a rapid, small scale production
of bacteriophage or plasmid DNA, e.g., as described by Maniatis
et al., supra, at p. 365-373.

In accordance with another aspect of this invention, there is provided a pharmaceutical preparation of AHF.  A
pharmaceutical preparation of human AHF produced in accordance
with this invention may be prepared for parenteral administration
in accordance with procedures well known in the art.

The pharmaceutical preparation for human use comprises
sterilized AHF recovered from transformed cells.  In addition to
the AHF polypeptide or sufficient portion thereof which produces
AHF activity, there may be included one or more acceptable
carriers therefor and optionally other therapeutic ingredients.
The carrier(s) must be "acceptable" in the sense of being
compatible with other ingredients of the preparation and not
deleterious to the recipient thereof.  The preparation may
conveniently be presented in unit dosage form and may be prepared
by any of the methods well known in the art of pharmacy.

The pharmaceutical preparation of this invention,
suitable for parenteral administration, may conveniently comprise
a sterile lyophilized preparation of the AHF polypeptide which
may be reconstituted by addition of sterilized solution to
produce solutions preferably isotonic with the blood of the
recipient.  The preparation may be presented in unit or
multi-dose containers, for example sealed ampoules or vials.

It should be understood that in addition to the sterile
AHF or solution thereof, the pharmaceutical preparation of this
invention may include one or more additional ingredients such as
diluents, buffers, binders, surface active agents, thickeners,
lubricants, preservatives (including anti-oxidants) and the like.
Gelatine, lactose, starch, magnesium sterate, micronized silica
gel, cocoa butter, talc, vegetabilic and animalic fats and oils,
vegetabilic rubber and polyalkylene glycol and other known
carriers for pharmaceuticals are all suitable for manufacturing
the pharmaceutical preparations of the present invention.
Preparations for parenteral use include an ampoule of a sterile
solution or suspension with water or other pharmaceutically
acceptable liquid as the carrier therefor, or an ampoule of sterile solid AHF for dilution with a pharmaceutically acceptable liquid.

The pharmaceutical preparation of the present invention is useful in the treatment of Hemophilia A. These preparations also provide an important tool in the in vivo as well as the in vitro study of the processes involved in clotting and in the study of the immunulogical and biological characteristics of the AHF molecule.

## Example 1. Protein Sequence Analysis

Porcine factor VIII:C was purified by Dr. David Fass according to published procedures, Knutsen and Fass (1982); Fass et al. (1982), supra. Amino acid sequence analysis is performed on a bovine thrombin digest product of the 76,000 dalton protein as described below.

Porcine AHF polypeptides bound to the anti-VIII:C monoclonal antibody column, can be sucessively eluted in two steps (Fass et.al. 1982). The two larger molecular weight species, 166,000 and 130,000 daltons, can be eluted with EDTA. The remaining polypeptide, having a molecular weight of about 76,000 daltons, can then be eluted with 50% ethylene glycol.

The 76,000 dalton protein is digested with thrombin after extensive dialysis in 50 mM Tris-HCl (pH 7.5), 0.15 M NaCl. Bovine thrombin digests are performed at room temperature for 60 minutes using 1 unit/ml of bovine thrombin, followed by the addition of another 1 unit/ml thrombin and incubation for an additional 60 minutes. The thrombin digestions are terminated by heating for 10 minutes at 90°C in 0.01% SDS. The major thrombin digest product of the 76,000 dalton protein is a polypeptide, 69,000 daltons (69 Kd).

Less than 1 μg of the 69 Kd polypeptide species described above is iodinated to serve as a radioactive marker after SDS gel electrophoresis, in accordance with the procedure of U. K. Laemmli, Nature, 227:680 (1970), the disclosure of which is

0182448

incorporated herein by reference. The polypeptide, dissolved in TAS buffer (50 mM Tris-acetate (pH 7.8), 0.1% SDS) is added to 100 μl of the same buffer containing 5 mCi of carrier-free iodine-125. 50 μl of 2.5 mg/ml chloramine T (Baker) in TAS buffer are added and the solution agitated for 1 minute. The reactions are stopped by adding 50 μl 2.5 mg/ml sodium metabisulfate in TAS buffer followed by 1 minute agitation. Labeled protein is separated from unincorporated I125 by chromatography using a small volume Sephadex G-25 M column (PD-10, Pharmacia). The column is pre-equilibrated with several column volumes of TAS buffer containing 2.5 mg/ml sodium iodide. The void volume is collected and protein integrity analyzed by SDS gel electrophoresis in accordance with the procedures of Laemmli, (1970), supra.

The radioactively labeled 69 Kd protein is added to its unlabeled counterpart for subsequent monitoring. The protein is then individually electrophoretically concentrated. The protein solutions are adjusted to 0.1% SDS, 10 mM dithiothreitol and Coomassie brilliant blue (Serva) are added to make the solution a very pale blue.

The solutions are then dialyzed briefly (1-2 hours) in TAS buffer using Spectrapore dialysis tubing (made by Spectrum Medical Industries, Inc., with molecular weight cut off at about 14,000). The dialyzed protein samples are then placed in an electrophoretic concentrator, of the design suggested by Hunkapiller, et al Meth. Enzymol., Enzyme Structures, Part I, 91:227 (1983), and electrophoretically concentrated in TAS buffer for 24 hours at 50 volts.

The 69 Kd AHF polypeptide, concentrated by the above procedure, is electrophoresed through an SDS-polyacrylamide gel in accordance with Laemmli, supra. The purified protein, identified by autoradiography of the radioactively labeled tracer polypeptide, is excised from the gel and electroeluted and concentrated as descibed by Hunkapiller et al., supra. The concentrated sample is suitable for direct amino acid sequence analysis using the gas phase sequenator as described in Hewick et al., J. Biol. Chem. 256:7990 (1981).

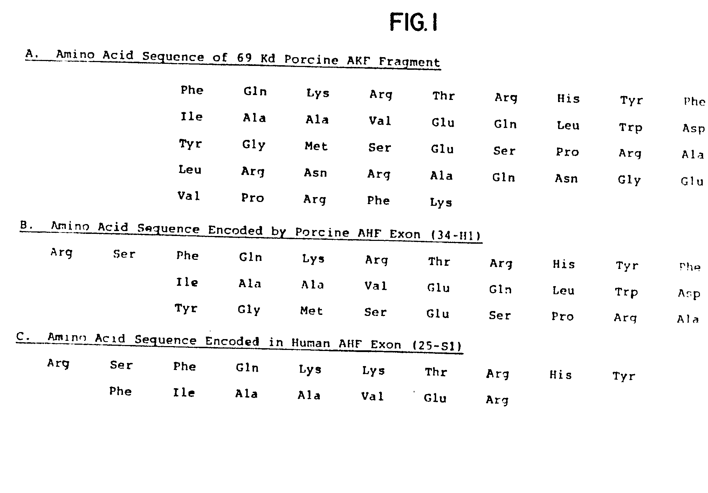

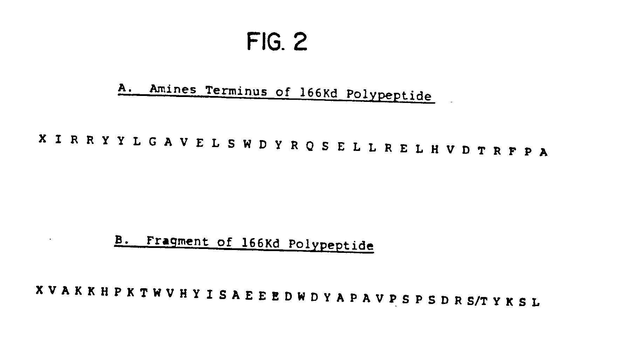

The amino terminal sequence extending from the 2-42 residue of the 69,000 dalton thrombin cleavage product is as depicted in Figure 1. The first residue "X" was not identifiable. The amino acid sequences of (A) the amino terminus and (B) a 40 kd bovine thrombin digestion product from the 166 kd AHF fragment noted by Fass et al., supra, are shown in Figure 2. The amino acid sequence of the amino terminus of the 76 Kd polypeptide is: X-Ile Ser Leu Pro Thr Phe Gln Pro Glu Glu Asp Lys Met Asp Tyr Asp Asp Ile Phe.

## Example 2

### Chemical Synthesis of Oligonucleotide Probes for a Porcine AHF Gene

#### (a) Pentapeptide Probe Pool

The partial amino acid sequence of a fragment of porcine AHF having been determined allows porcine AHF oligonucleotide probes to be designed and synthesized. From the genetic code (Table 1) it is possible to predict the gene sequence that codes for this sequence of amino acids. Because the genetic code is degenerate, there are more than one possible DNA coding sequences for each amino acid sequence. Accordingly, a plurality or pool of complementary oligonucleotide probe sequences are provided for a region of the AHF molecule which required only a reasonable number of oligonucleotides to ensure the correct DNA sequence. Such regions are selected by searching for tracks of five to eight contiguous amino acids which have the lowest degeneracy. After the region is selected, a pool of oligonucleotides is synthesized, which would include all possible DNA sequences which could code for the five to eight amino acids in the selected region.

In the 69,000 dalton thrombin-cleavage fragment of porcine AHF there is a pentapeptide sequence running from the 18th through the 22nd amino acid from the amino terminus, which could be coded for by up to 16 different DNA sequences, each having five codons, for a length of 15 nucleotides.

|  | 18 |  | 20 |  | 22 |
|---|---|---|---|---|---|
|  | Trp - | Asp - | Tyr - | Gly - | Met |
| Possible mRNA Sequences | UGG | GAU | UAU | GGU | AUG |
|  |  | or | or | or |  |
|  |  | GAC | UAC | GGC |  |
|  |  |  |  | or |  |
|  |  |  |  | GGA |  |
|  |  |  |  | or |  |
|  |  |  |  | GGG |  |

The probes are made by synthesizing a limited number of mixtures of oligonucleotides with two to eight or more oligonucleotides per mixture. These mixtures are referred to as pools. Enough pools are made to encompass all possible coding sequences.

These oligonucleotides can be synthesized manually, e.g., by the phospho-tri-ester method, as disclosed, for example in R. L. Letsinger, et al., J. Am. Chem. Soc. 98:3655 (1967), the disclosure of which is incorporated by reference. Other methods are well known in the art. See also Matteucci and Caruthers, J. Am. Chem. Soc. 103:3185 (1981), the disclosure of which is incorporated by reference.

Preferably, however, the synthetic oligonucleotide probes for the desired polypeptide sequences are prepared by identical chemistry with the assistance of the completely automatic Applied Biosystems DNA synthesizer, Model 380A, as indicated above.

The oligonucleotides thus prepared can then be purified on a reverse HPLC column, as described by H. Fritz, et al., Biochemistry, 17:1257 (1978), the disclosure of which is incorporated herein by reference. After detritylation with 80% HOAc, the resulting oligonucleotide is normally pure and can be used directly as a probe. If there are any contaminants, the synthetic DNA can be further purified on the same HPLC column, preferably using a slightly different gradient system.

Oligonucleotides are labelled, e.g. by using [ $-32$P]ATP and T4 polynucleotide kinase, and their sequence checked either by two-dimensional homochromatography as described by Sanger et al., PNAS U.S.A. 70:1209 (1973) or by the Maxam-Gilbert method, Meth. Enzymology, 65:499 (1977), the disclosures of both of which are incorporated herein by reference.

(b) Forty Five Nucleotide Probes

A unique aspect of the present invention has been the use of oligonucleotide probes to screen a genomic DNA library for the AHF gene or fragments thereof. While oligonucleotides have been used for screening of cDNA libraries, see, e.g. M. Jaye, et al, Nucleic Acids Research 11:2325 (1982), the disclosure of which is incorporated herein by reference, genomic libraries have previously been screened successfully only with cDNA probes, i.e. probes which were generated only after the tissue source of the mRNA for a described protein had been identified and utilized to generate a cDNA clone which precisely matched the DNA sequence of the gene sought by the genomic search.

In the present case, it has been shown possible to use oligonucleotides to identify gene segments in a genomic library which code for the amino acid sequence of the proteins of interest, and identification of such gene segments provides an exact probe for use in mRNA, cDNA or further genomic screening techniques.

Preferably, as here, oligonucleotides corresponding to at least two segments of the amino acid sequence of the protein of interest are utilized. Preferably at least one of the oligonucleotide probes is used in the form of one or more pools of oligonucleotides which in the aggregate include every possible DNA sequence which could code for the amino acid sequences selected. Preferably one relatively short probe, e.g. 11 to 25

0182448

nucleotides, preferably 15 to 20 nucleotides is utilized in conjunction with a relatively long probe, e.g. 30 to 200 nucleotides, preferably 40 to 50 nucleotides. The second probe can be used for confirmation, and is not always necessary for identification of the DNA segment. Preferably at least one of the probes, and more preferably the longer of the probes is designed in accordance with the Rules 1 to 4 described below.

Rule 1. Codon Preference In the absence of other considerations, the nucleotide sequence was chosen which matched prevailing or similar sequences in similar mammalian genes. See Mechanisms of Ageing Dev., 18:(1982).

Rule 2. Advantageous GT Pairing The nucleotide G, in addition to bonding to its compliment C, can also form weak bonds with the nucleotide T. See K. L. Agarwal et al., J. Biol. Chem. 256:1023 (1981). Thus, faced with a choice of G or A for the third portion of an ambigious codon, it is preferable to choose G, since if the resulting hybridization would occur even if the actual nucleotide in the position is a T, rather than a C, the hybridization would still be stable. If an A were chosen incorrectly, the corresponding A:C incompatability could be enough to destroy the ability of the probe to hybridize with the genomic DNA.

Rule 3. Avoidance of 5'CG Sequences

When selecting from among the possible ambiguities, select those nucleotides which will not contain the 5'C$_p$G sequence, either intra codon or inter codon.

Rule 4. Mismatch Position In choosing the codon sequences to use, consideration was given to the postulation that mismatches near the ends of the molecule do not adversely affect the stability of the hybridization as do mismatches near the center of the molecule. Thus, for example, where substantial doubt occurred concerning the sequence of a particular codon, and that codon was close to the center of the probe, the tendency was to test a pool of possible nucleotide sequences for that codon,

whereas codon positions near the ends of the probe were more likely to be subject to determinations on the basis of codon preference.

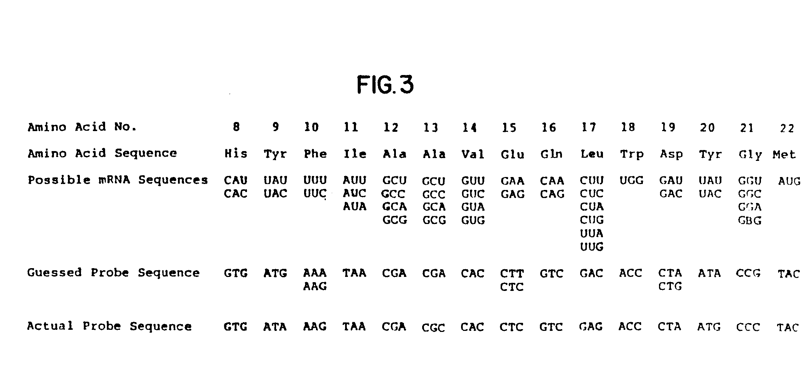

The chosen sequence for the 45-mer probes, as well as the amino acid sequences, the possible DNA sequences, and the "Actual Probe Sequence", i.e. the complement of actual coding strand determined for the AHF exon, as shown in Figure 3.

Thus, out of nucleotide positions involving choices, three were covered by using pools containing both possible nucleotide alternatives, five were predicted correctly, one was predicted in a way to maintain neutrality though incorrect, and four others were in error.

Despite the approximately 11% mismatch (5/45) the pool of 45-mer oligonucleotides are adequate to strongly identify a porcine AHF gene fragment, as described below.

### Example 3. Screening of Porcine Genomic Library

A porcine genomic library is constructed using the bacteriophage vector Lambda J1. Lambda J1 is derived from L47.1 (Loenen et al., Gene 20:249 (1980)) by replacement of the 1.37 kb and 2.83 kb Eco RI-Bam HI fragments with a 95 bp Eco R1-Hind III-Xba I-Bgl II-Bam HI polylinker. The 6.6 kb Bam HI fragment is then present as a direct repeat in reverse orientation relative to L47.1. The cloning capacity for Bam HI fragments is 8.6 - 23.8 kb. Bam HI cleaved porcine DNA (prepared as described by Piccini et al., Cell, 30:205 (1982)) is phenol extracted, ethanol precipitated and concentrated by centrifugation in a Microfuge. 0.67 µg of Bam HI porcine DNA is ligated to 2 µg of Lambda J1 Bam HI "arms", prepared as described in Maniatis, et al., supra, pp 275-279, in a volume of 10 µl ligation buffer with 10 units T4 DNA ligase (Maniatis et al., supra, p 474). The ligated DNA is packaged and plated as described in Maniatis et al., supra, p 291.

Approximately $4 \times 10^5$ pfu are plated on E. coli stain C600 on 15 cm plastic petri plates containing NZCYM agarose, at

0182448

8,000 pfu/plate. These recombinant phage are screened by the method of Woo (1979) using the 45-mer probe described above, radioactively labeled with $32_P$ as described above, as a probe. Filters are then hybridized in 5xSSC, 5x Denhardt's, 0.1% SDS, and 5 x $10^6$ cpm/ml probe at 45° C for 16 hours, washed in 5 x SSC, 0.1% SDS at 50° C and subjected to autoradiography using intensifying screens (DuPont Lightning-Plus). Autoradiography reveals numerous phage which hybridized, to varying degrees, with the 45-mer. The filters are then denatured in 0.5M NaOH, neutralized in 1.0M Tris pH7.5, 1.5M NaCl and hybridized to the 15-mer as described for the 45-mer except the hybridization and washing temperature is 37° C. One phage which hybridized to both probes is picked from the original plate and 100 pfu plated and the plaques screened as described above using the 15-mer as probe.

A positive phage, named PB34, is picked as a plug and used to make a plate lysate as described in Maniatis et al, supra, pp 65-66. A small-scale isolation of PB34 DNA is achieved using the procedure described in Maniatis et al., supra, pp 371-372. 10μl of this DNA was cut with the restriction enzyme Hae III and then phosphatased using calf alkaline phosphatase (Boehringer-Mannheim). After phenol extraction, 20 ng of Sma I cut M13mp8 DNA is added, the solution is made 0.2M NaCl and nucleic acid precipitated by the addition of 2 volumes ethanol. Precipitated DNA is pelleted by centrifugation and redissolved in 2 μl of a ligation mixture and the DNA is ligated for 30 minutes at 23° C, diluted to 50 μl with ligase buffer and ligated an additional 3 hours. 5 μl of this reaction is used to transform E. coli strain JM101/TG1.

Cells are made competent for transformation by growing to an $0.D._{600}$ of 0.5 at 37° C in 50 ml SOBM media (SOBM is 2% tryptone, 0.5% yeast extract, 0.1M NaCl, 0.11g KOH per liter, 20mM MgSo4). Cells are pelleted by centrifugation at 2500 rpm for 10 minutes at 4° C. The cells are resuspended in 3.5 ml

0182448

100mM RbCl, 45mM MnCl2, 50mM CaCl2, 10mM potassium MES pH 6.4
(MES = methylethane sulfonic acid). 200ul of competent cells are
transformed with the DNA contained in 5 ml of the ligation
reaction at 0° C for 30 minutes. The cells are then heat-shocked
at 42° C for 90 seconds after which 4 ml of 0.8% agarose/SOBM
containing 100 ul stationary JM101/TG1 cells are added and plated
on 10 cm SOBM agar petri plates.

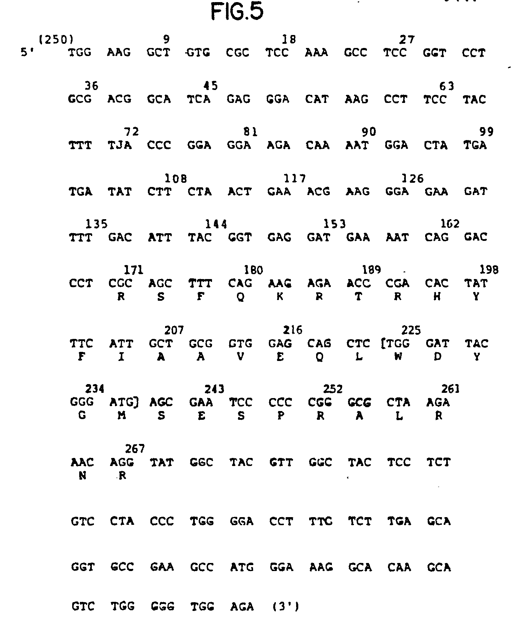

A subclone, containing a Hae III fragment from PB34,
hybridizing to the 15-mer is identified by screening using the
procedure of Benton and Davis, supra. This clone is isolated and
prepared for use as a template. The DNA sequence of the Hae III
fragment present in this clone, designated as 34-H1, is shown in
Figure 5. M13 template DNA is prepared by growing 1.5 ml of
infected cells 5 hours at 37° C. Cells are pelleted by
centrifugation for 10 minutes in a Beckman microfuge. 1.0 ml of
supernatant (containing virus) is removed and 200 ul of 20%
polyethylene glycol, 2.5 M NaCl is added. This sample is then
incubated at room temperature for 15 minutes followed by
centrifugation for 5 minutes in a Beckman microfuge. The pellet
is dissolved in 100 ul TE, 7.5 ul of 4M NaCHCOO pH 4.5 is added
and the sample extracted twice with a 1:1 mixture of
phenol-chloroform and once with chloroform. The single-stranded
phage DNA is then precipitated by the addition of 2 volumes of
ethanol. Precipitated DNA is pelleted by centrifugation in a
Beckman microfuge and dissolved in 30 ul 1mM Tris pH 8.0, 0.1 mM
EDTA. The DNA sequencing is performed by the dideoxy chain
termination technique, see, e.g. Sanger et al., PNAS U.S.A.,
74:5463 (1977), utilizing the 15-mer as a primer. The sequence
observed, which is included in the sequences represented in Figs.
4 and 5, confirmed the subclone as containing a porcine AHF exon
as it includes the identical 14 amino acids encompassed by the
region phenylalanine$_2$ to glutamine$_{15}$ in the amino terminal
sequence of the 69K fragment represented in Figure 1. Further
confirmation came from sequencing from the 5' end of the Hae III

0182448

insert in the 34-Hl vector, by priming with the "Universal primer" (Bethesda Research Labs) at a point adjacent the polylinker in that vector. Also, the insert from this clone, named 34-Hl, was recloned into Ml3mp9 by restricting the DNA with Eco RI and Hind III, phosphatasing with calf alkaline phosphatase, and ligating to Eco RI, Hind III cleaved Ml3mp9 DNA. This clone, which contains an inversion of the Hae III segment relative to the universal primer, was also sequenced as described above. The resulting sequence data for all of insert 34-Hl is shown in Figure 5. This sequence confirms that this subclone contains an exon of the porcine AHF gene that could encode, from nucleotides 169 to 267, at least the thirty amino acids from the phenylanine$_2$ through arginine$_{31}$ of the 69K fragment (Fig. 1).

It appears likely that arginine$_{31}$ borders an intron because termination codons can be found downstream from nucleotide 267 (Fig. 5) in all three reading frames, and a sequence similar to the consensus 5' splice site sequence is also found in that region between nucleotides 266-267. Further, the amino acid sequence which would be encoded by the downstream DNA differs completely from that observed in the 69 Kd fragment of porcine AHF.

PB34 DNA was cut with Bam HI and electrophoresed through an agarose gel and the bands visualized by ultraviolet light after staining the gel in 5 µg/ml ethidium bromide. Three inserts of approximately 6.6 kb, 6.0 kb, and 1.8 kb were observed. The DNA in the gel was transferred to nitrocellulose as described in Maniatis et al., supra, pp 383-386. Hybridization of the filter to the 15-mer and autoradiography were performed as described above. Autoradiography revealed that the 6.0 kb band contained an AHF gene fragment which hybridized to the 15-mer probe.

Thus, for the first time, a section of the gene coding for porcine AHF had been isolated and identified. The bacteriophage lambda recombinant clone PB 34 is on deposit at the

0182448

American Type Culture Collection under Accession Number ATCC 40087.

Example 4.  Location of Human AHF Gene

The human genomic library described by Maniatis et al., Cell, 15:687 (1978) is screened for the human AHF gene by infecting E. coli strain LE392 (publicly available) with 6x10⁵ pfu and plating on 15cm NZCYM agar plates at a density of 20,000 pfu/plate.  These phage are screened using the procedure of Benton and Davis, supra with the 6.0 kb porcine AHF fragment described in Example 3, labeled with ³²P by nick translation, as the probe.  A phage exhibiting a strong hybridization signal is picked and plated at about 100 pfu/10cm plate and screened in duplicate as described above using the radioactively labeled 45-mer as one probe and the 6.0 kb Bam HI fragment of PB34 as the other.  A phage, named HH25, which hybridizes to both probes is identified, a plate stock made and rapid prep DNA prepared as described above.  The phage DNA is cut with Sau3A I, phosphatased with calf alkaline phosphatase phenol extracted, and co-precipitated with 20 ng of Bam HI cut M13 mp8 DNA.  Precipitated DNA is pelleted by centrifugation and redissolved in 2 µl ligase buffer containing T4 DNA ligase.  Ligation is performed for 2 minutes at 16° C, diluted to 50 µl in ligase buffer containing T4 DNA ligase and incubated an additional 3 hours at 16° C.  5 µl of this reaction mixture is used to transform E. coli strain JM101/TG1 as described in Example 3 above.

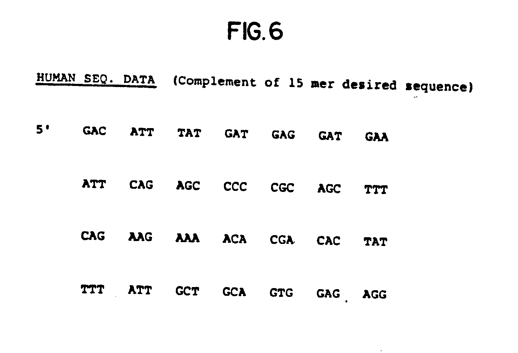

The plaques are screened using the Benton and Davis procedure and probing with radioactively labeled 15-mer.  A phage plaque, named 25-S1, exhibiting hybridization is isolated and single-stranded phage DNA prepared for use as a DNA sequencing template as described above.  Sequencing is performed using the dideoxy chain termination technique described by Sanger et al., supra, utilizing the 15-mer as primer, and gives the information strand DNA sequence shown in Figure 6. 84 nucleotides sequenced
in this manner demonstrated 85% homology with the homologous
region of porcine AHF. There is also only one amino acid
difference between the porcine AHF 69K region 2-16 as shown in
Figure 1 and the corresponding region which was deduced from the
human nucleotide sequence of Figure 6. This high degree of
homology shows that the DNA of the recombinant phage HH-25
emanates from the AHF gene.

Thus, for the first time, an exon for the human AHF gene
has been isolated and identified. A bacteriophage lambda
recombinant HH 25 is on deposit at the American Type Culture
Collection under the Accession Number ATCC 40086.

### Example 5  Identifying Cells Actively Transcribing AHF

The porcine and human AHF exons described above are
useful for a variety of functions, one of which is as a screening
agent which permits identification of the tissue which is the
site of synthesis of AHF in vivo. A number of screening methods
are available, based on the use of the exon as an exact
complement to the mRNA which is produced during the course of
natural expression of AHF. In the screening procedures, tissue
from various parts of the body is treated to liberate the mRNA
contained therein, which is then hybridized to a DNA segment
containing the exon for AHF, and if a molecule of mRNA does
hybridize to that exon, the tissue which is the source of that
mRNA is the source of AHF.

### 1. Screening Procedure

Porcine or human tissue from various organs, including
kidneys, liver, pancreas, spleen, bone marrow, lymph nodes, etc.,
is prepared by guanidine hydrochloride extraction as described by
Cox Methods Enzymol., 12B:120 (1968), the disclosure of which is
incorporated herein by reference, with some modifications.
Briefly, tissue is explanted into 8 M guanidine hydrochloride (or
4M guanidine isothiocyanate as proposed by Chirgwin, et al.,

Biochemistry 18: 5294 (1979), the disclosure of which is
incorporated herein by reference, see also Maniatis, et al.,
supra, at 189 et seq.), 50 mM Tris (pH 7.5), 10 mM EDTA and
homogenized in an Omnimixer (Sorvall) at top speed for 1 minute.
The homogenate is clarified at 5000 rpm for 5 minutes in a Sorval
HB-4 rotor and the RNA is precipitated by the addition of 0.5
volumes of ethanol. The RNA is dissolved and precipitated 3 more
times from 6 M guanidine hydrochloride before being dissolved in
H2O.

Messenger RNA from this pool is enriched by
chromatography on oligo (dT) cellulose (Collaborative Research).

This mRNA is then subjected to electrophoresis through an
agarose gel containing formaldehyde, as described by Maniatis et
al., supra, at 202-3. The mRNA in the gel is then transferred to
a nitrocellulose filter (Maniatis et al., supra, at 203-4).

The thus obtained mRNA is hybridized with the
radiolabeled porcine or human exon DNA obtained as described
above, and the existence of hybrids detected by autoradiography.
A radioactive signal indicates that the tissue source of the mRNA
is a source of synthesis of AHF in the body.

Alternatively, mRNA can be screened using the S1
protection screening method.

S1 nuclease is an enzyme which hydrolyzes single stranded
DNA, but does not hydrolyze base paired nucleotides, such as
hybridized mRNA/DNA. Thus the existence of a radioactive band
after acrylamide gel electrophoresis and autoradiography shows
that the single stranded DNA corresponding to the AHF exon has
been protected by the complementary mRNA, i.e. the mRNA for AHF.
Thus the tissue which was the source of that mRNA is a site of
synthesis of AHF in vivo. That tissue can be used as a source
for AHF mRNA. This method of screening can be somewhat more
sensitive than screening method described above.

A probe consisting of single stranded radioactively
labeled DNA complementary to the mRNA is synthesized using the universal primer of M13 to prime DNA synthesis of the porcine
genomic subclone 34-H1.  The reaction is performed in a 100 $\mu$l
solution of 50 mM Tris pH 7.4, 5 mM MgCl$_2$, 1 mM
2-mercaptoethanol, 50 mM NaCl, 40 $\mu$M dGTP, dTTP, dCTP, 60 $\mu$Ci of
$^{32}$P-dATP (400Ci/mmole), 10 ng universal primer, 200-400 ng of
34-53 template DNA, and the Klenow fragment of DNA polymerase I
(E. coli).  The reaction is incubated at 23° C for 60 minutes, 10
minutes at 70° C, 50 units of PstI added and incubated an
additional 60 minutes.

The reaction is terminated by phenol/chloroform
extraction, NaCl added to 0.2M and then precipitated with two
volumes 100% ethanol.  The precipitated DNA is pelleted by
centrifugation, redissolved in 20% sucrose, 50mM NaOH, 0.1%
cresol green and then electrophoresed through 2% agarose in 50 mM
NaOH, 10 mM EDTA.  The resulting single stranded fragment is
localized by autoradiography, the band excised and DNA isolated
by electroelution in dialysis tubing.

Sample mRNA is prepared from liver, spleen, etc. tissue
by the guanadine hydrochloride method described above.

The probe is then hybridized to sample mRNA (obtained
from the oligo (dT) chromatography enriching step) in 50%
formamide, 0.4M NaCl, 40 mM PIPES [piperazine-N,N'
bis(2-ethanesulfonic acid)] pH 6.5, 1 mM EDTA, 5-50 $\mu$g mRNA, 2 $\mu$g
labeled DNA in a volume of 15 $\mu$l.  The hybridization is
terminated by the addition of 200 $\mu$l cold S1 nuclease buffer
(0.25 M NaCl, 0.3 M NaCH$_3$COO[pH 4.5], 1 mM ZnSO$_4$, 5% glycerol,
and 1000 units S$_1$ nuclease.  The reaction is incubated at 45° C
for 30 minutes.  Samples are phenol extracted, ethanol
precipitated with 10 $\mu$g yeast tRNA carrier and subjected to
analysis by electrophoresis through 5% polyacrylamide sequencing
gels as described in Maxam-Gilbert, PNAS USA, 74:560 (1977).

Example 6  Use of AHF Exon DNA to Obtain AHF mRNA From Tissue

Once a tissue which is a source of mRNA is identified,

0182448

AHF mRNA from that tissue is extracted and used to construct a cDNA library. The cDNA library is then utilized for identifying and constructing a full length cDNA clone which encodes the amino acid sequence for AHF, without the introns contained in the genomic clone, as described below. Thereafter, the cDNA which encodes the AHF protein is inserted into an appropriate expression vector, in an appropriate host, for expression of AHF.

Since human AHF is in great demand for treatment of hemophilia and other uses, the preparation of human AHF cDNA is described.

### 1. Obtaining mRNA for Human AHF

mRNA from the human tissue responsible for AHF synthesis is prepared by the guanidine hydrochloride extraction method as described by Cox and modified by Chirgwin, et al. as disclosed above.

Further fractionation of mRNA obtained from the oligo (dT) cellulose chromatography column is obtained by sedimenting on 5-20% sucrose gradients containing 10 mM Tris-HCl (pH 7.4), 1mM EDTA and 0.2% SDS by centrifugation for 24 hours at 22,000 rpm in a Beckman SW28 rotor. Fractions (1.0 ml) are collected, sodium acetate was added to 0.2M, and the fractions are ethanol precipitated twice before dissolving in water. The size distribution of the fractionated RNA is determined by electrophoresis through 1.4% agarose gels containing 2.2 M formaldehyde.

mRNA sedimenting with an S (Svedberg) value greater than 28 is pooled for the synthesis of double stranded cDNA. 10 μg of this RNA is denatured at room temperature in 10 μl of 10 mM methylmercuryhydroxide. 140 mM 2-mercaptoethanol is added to inactivate the methylmercuryhydroxide. The RNA is then diluted to 50 μl containing 140 mM KCl, 100 mM Tris-HCl (pH 8.3 at 42°C), 1 mM of each deoxynucleotide triphosphate, 200 μg/ml oligo(dT)12-18, 10 mM MgCl₂, and 0.1 μCi 32P-dCTP/ml. These reactions are performed at 42°C for 1 hour after the addition of

3 μl of 17 units/μl AMV reverse transcriptase (Life Sciences).
The reaction is terminated by the addition of 0.25 M EDTA (pH
8.0) to 20 mM. The resulting mixture is extracted once with an
equal volume of phenol/chloroform (1:1) followed by one
chloroform extraction. The sample is then chromatographed on a 5
ml Sepharose CL-4B column (Pharmacia) equilibrated in 10 mM
Tris-HCl (pH 8.00, 100 mM NaCl, 1 mM EDTA. The void volume is
collected and the nucleic acids (including any RNA/cDNA hybrids)
are precipitated by the addition of 2.5 volumes of ethanol.

Preferably in conjunction with the above procedures an
AHF exon oligonucleotide segment is also used in place of oligo
dTr to prime the reverse transcription, as described in Ullrich
et al., Nature, 303:821 (1983).

The RNA-cDNA hybrids are dissolved in 35 μl of deionized
H2O, made 100 mM potassium cacodylate (pH 6.8), 100 μM dCTP, 1 mM
2-mercaptoethanol, 1 mM cobalt chloride and enzymatically
"tailed" by the addition of 10 units of deoxytidyl terminal
transferase (pH Biochemicals) and incubating the reaction for 30
seconds at 37°C. The reaction is terminated by adding 0.25 M
EDTA to 10 mM. Tris-HCl (pH 8.0) is added to 300 mM and the
sample extracted once with an equal volume of phenol-chloroform
(1:1) and then with an equal volume of chloroform. Nucleic acids
are precipitated from this product by the addition of 2.5 volumes
of ethanol.

The dC tailed hybrid molecules are then annealed with 170
μg/ml oligo(dG)14-18 cellulose in 10 mM KCl, 1 mM EDTA for 10
minutes at 43°C and then an additional 10 minutes at 23°C. This
reaction product is then diluted to 100 μl containing 100 mM
ammonium sulfate, 1 mM 2-mercaptoethanol, 100 mM MgCl2, 100 μg/ml
bovine serum albumin (Sigma, Cohn fraction V), and 100 μM
nicotinamide adenine dinucleotide. Second strand cDNA synthesis
is initiated by the addition of 1 unit RNase H (P-L
Biochemicals), 1 unit of E. coli DNA ligase, and 10 units of DNA
polymerase I and incubated at 16°C for 12 hours.

The sample is then chromatographed over Sepharose CL-4B as described above. Double stranded DNA is ethanol precipitated and dC tailed as described for the RNA-cDNA hybrid tailing.

### 2. Screening for Human AHF DNA

dC tailed double stranded cDNA obtained as described above is annealed with an equimolar amount of dG tailed pBR322 (New England Nuclear) in 10 mM Tris-HCl (pH 8.0), 1 mM EDTA, 100 mM NaCl at 37° for 2 hours. The annealed chimeric molecules are then frozen at -20°C until use in bacterial transformation.

Bacterial transformation is done using the MC1061 strain of E. coli(source). Cells (50 ml) are grown to an optical density of 0.25 at 600 nm. Cells are concentrated by centrifugation at 2,500 rpm for 12 minutes, washed in 10 ml of sterile 100 mM $CaCl_2$, and again pelleted by centrifugation as described above. Cells are resuspended in 2 ml of sterile 100 mM $CaCl_2$ and kept at 4° for 12 hours. The annealed chimeric molecules are incubated at a ratio of 5 ng of double stranded cDNA per 200 µl competent cells at 4°C for 30 minutes. The bacteria are then subjected to a two minute heat pulse of 42°C. 1.0 ml of L-broth is then added and the cells incubated for 1 hour at 37°. Cells are then plated onto LB-agar plates containing 5 µg/ml tetracycline.

Human AHF clones are identified using the colony hybridization procedure of Grunstein and Hogness PNAS U.S.A. 72:3961 (1975), the disclosure of which is incorporated herein by reference. The cDNA library is plated onto a nitrocellulose filter (Schleicher and Schuell) overlaying L-broth/ 5 µg/ml tetracycline agar plates. Colonies are grown overnight at 37°C and then the filter placed on sterile Whatman 3 M paper. A pre-moistened nitrocellulose filter is then pressed against the master filter and the filters keyed using an 18 gauge needle. The replica filter is then grown on LB-tetracycline plates at 37°C until the colonies reached a diameter of 1-2 mm. The filters are then transferred to LB plates containing 150 µg/ml

chloramphenicol and incubated at 37°C for 16-24 hours.

Filters are then removed and placed onto Whatman 3 M paper saturated with 0.5 M NaOH for 5 minutes at room temperature.  Filters are then neutralized by placing onto Whatman 3 M saturated with 1 M Tris-HCl (pH 7.5), 1.5 M NaCl, and then Whatman 3 M saturated with 2x standard saline citrate (SSC). SSC (1x) is 0.15 M NaCl, .015 M sodium citrate.

Filters are air dried and baked in vacuo at 80°C for 2 hours.  Prehybridization of filters is done at 65°C for 30 minutes in 10 mM Tris-HCl (pH 8.0), 1 mM EDTA, .1% SDS followed by 30 minutes in 7x SSC, 5x Denhardt's (1x Denhardt's is 0.02% polyvinylpyrollidine, 0.02% ficoll, 0.02% bovine serum albumin), 100 ug/ml denatured salmon sperm DNA, and 0.1% SDS.  32P-labelled human exon DNA, prepared as described above, are added to 106 cpm/ml and the hybridization performed 12-16 hours at 37°C. Filters are then washed in several changes of 7x SSC, .1% SDS for 1-2 hours at 37°C.  Filters are then air-dried and subjected to autoradiography with Kodak XAR film and a Dupont Lightning Plus intensifying screen.

Those colonies showing a hybridization signal above background are grown in L-broth containing 50 ug/ml tetracycline for rapid prep purification of plasmid DNA.  Plasmid DNA is purified by the method of Holmes et al., Anal. Biochem., 114:193 (1981), the disclosure of which is incorporated herein by reference.  An aliquot of this DNA is cleaved with restriction endonuclease Pst 1 and the fragments electrophoresed through 1% agarose/TBE gels and blotted according to the procedure of E. Southern, J. Mol. Biol. 98:503 (1975), Methods Enzymol. 69:152 (1980), the disclosure of which is incorporated herein by reference.  The nitrocellulose filters are hybridized with radiolabeled human AHF exon DNA as described for colony hybridization.  Those plasmids which contained Pst I inserts hybridizing to the AHF exon DNA are used for DNA sequencing analysis.

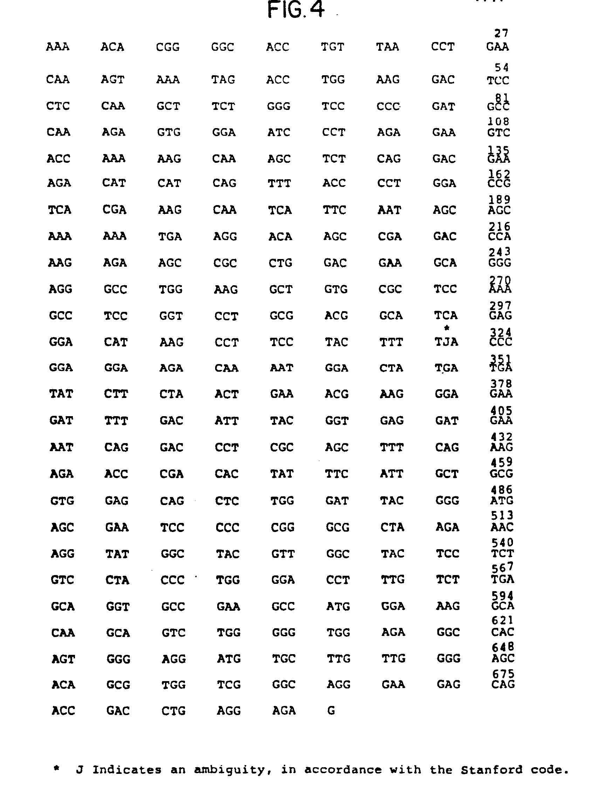

For sequencing, plasmid DNA, (purified from 0.75 ml of culture by the procedure of Holmes, et al., supra), is digested to completion with the restriction endonuclease Sau 3aI. The resulting DNA segment, identified as 34-S1, has a DNA sequence which is depicted in Figure 4. The DNA is ethanol precipitated after extraction with phenol-chloroform and redissolved in 10 µl TE (10 mM Tris-HCl pH 8.0, 1 mM EDTA). 5 µl of the DNA solution is ligated with 20 ng of Bam Hl cleaved M13 mp 9 replicative form DNA in 100 ul of 50 mM Tris-HCl (pH 7.4), 10 mM MgCl$_2$, 10 mM dithiothreitol, 1 mM ATP and an excess of T4 DNA ligase. Ligations are done at 15°C for 2-4 hours.

5 µl of the ligation reactions are used to transform 200 ul of E. coli strain JM101/TG1 as described above. Recombinants are identified as white plaques when grown on LB-agar plates containing X-gal as an indication for beta-galactosidase activity as described by Davies, et al., J. Mol. Biol. 36:413 (1968).

Recombinant phage harboring sequences hybridizing to the human exon are identified by the procedure of Benton, et al., Science 196:180 (1977), the disclosure of which is incorporated herein by reference, using radioactively labelled human AHF exon as a probe. Plaques showing a hybridization signal are picked and grown in 1.5 ml cultures of L-broth. Single-stranded phage DNA prepared from these cultures is used as template in oligonucleotide-primed DNA synthesis reactions. Sequencing is done using the dideoxy chain-termination procedure, see, e.g., Sanger et al., PNAS U.S.A. 74:5463 (1977).

Human AHF recombinants are identified by comparing their nucleotide sequence with that which is known from the human exon sequence of human AHF.

### 3. Porcine AHF mRNA

Human AHF recombinants are used to screen a porcine tissue library constructed exactly as described for the human tissue cDNA library. Prospective porcine AHF recombinant DNA clones are identified by the Grunstein-Hogness procedure using

the porcine $^{32}$p labeled exon fragment as a probe as described above. The probe is the porcine AHF exon segment labelled with 32P by nick-translation as described by Rigby et al., J. Mol. Biol., 113:237 (1977), the disclosure of which is incorporated herein by reference.

Colonies exhibiting hybridization signals are grown for rapid prep plasmid purification purposes as described above. Plasmid DNA is cleaved with the restriction endonuclease Pst 1, electrophoresed through 1% agarose/TBE gels, and blotted according to the procedure of E. Southern (1975). The blots are hybridized with nick-translated porcine AHF recombinant DNA labelled with 32P.

Full-length clones may be constructed in a conventional manner, such as by ligation of DNA fragments from overlapping clones at restriction enzyme sites common to both clones as is well known in the art ("gene walking").

## 4. Identifying full-length clones from steps 2 or 3

The distance between the 5' end of an existing clone and the 5' end of the mRNA can be analyzed by the primer-extension technique described in Agarwal et al., "J.B.C." 256(2):1023-1028 (1981) utilizing an oligonucleotide primer whose sequence comes from the 5' (amino-terminal) region of the existing AHF clone. If gels developed using this procedure show more than one transcript one should consider the most intense band as representing the full mRNA transcript.

There are, however, many mRNAs which contain a long region of 5' untranslated sequence. Thus, expression of human AHN DNA may not be contingent upon the acquisition of a complete cDNA clone. For example, a clone may be obtained which by DNA sequencing analysis demonstrates the existence of a methionine codon followed by a sequence which is analogous to or identical to a known eucaryotic protein secretion signal and which is in frame with the remaining codons. Transformation and expression should be conducted for a clone which contains the met-secretion signal sequence, which is of the expected size and which contains a poly (T) 3' terminus.

### 5. Alternative Procedure

As an alternative to the methods in Sections 1-3 described above, it is preferred that the cDNA clones for porcine or human AHF be identified using a bacteriophage vector in the following manner.

mRNA from human fetal liver tissue responsible for AHF synthesis was prepared by the guanidine hydrochloride method as described by Cox and modified by Chirgwin, et al. as set forth above in Example 5, Section 1. First strand cDNA was synthesized from 10 µg of polyA+ fetal liver RNA by the procedure described in Example 6, Section 1, supra. Specifically, 10 µg of this RNA was denatured at room temperature in 10 µl of 10 mM methylmercuryhydroxide. 140 mM 2-mercaptoethanol was added to inactivate the methylmercuryhydroxide. The RNA was then diluted to 50 µl containing 140 mM KCl, 100 mM Tris-HCl (pH 8.3 at 42 °C), 1 mM of each deoxynucleotide triphosphate, 200 µg/ml olio(dT)12-18, 10 mM $MgCl_2$, and 0.1 µCi 32p-dCTP/ml. These reactions were performed at 42°C for 1 hour after the addition of 3 µl of 17 units/µl AMV reverse transcriptase (Life Sciences).

For the first strand synthesis of a primer-extended library, 200 picomoles of a unique complimentary 38mer was included in the $CH_3HgOH$ denaturation step, and after 10 minutes at 23°C, the reaction was made 140mM beta-mercaptoethanol, 0.7M KCl, 1mM EDTA, 20mM Tris-HCl (pH 8.3 at 42°), 1 unit/ul of RNasin (Biotec) and incubated at 50°C for 2 minutes and at 42°C for 2 minutes. This was then diluted to 50 µl containing 140 mM KCl, 100 mM Tris-HCl (pH 8.3 at 42°C), 1 mM of each deoxynucleotide triphosphate, 10 mM $MgCl_2$, and 0.1 µCi 32p-dCTP/µl. The reaction was incubated at 42 °C for 1 hour after the addition of 3 µl of 17u/µl AMV reverse transcriptase.

After first strand synthesis the reactions were diluted to 150ul containing 10mM $MgCl_2$, 50mM Tris pH 7.4, 5mM

0182448

2-mercaptoethanol, 7.5mM NH4SO4, 250 uM of each deoxynucletide triphosphate, and second strand synthesis initiated by the addition of 1 unit of RNase H (E. coli) and 45 units of DNA polymerse I. Reactions were incubated at 16°C for 8 hours and then terminated by the addition of EDTA to 20mM and brought to a final volume of 200µl with H2O. EcoRl methylation was then performed by addition of S-adenosyl methionine to 50uM and 40 units of EcoRl methylase. These reactions were incubated for 1 hour at 37°C, terminated by phenol-chloroform extraction, and chromatographed using Sephadex G50 equilibrated in 10mM Tris-HCl (ph 8.0), 1mM EDTA, .1M NaCl. The void volume was pooled and precipitated by ethanol addition.

cDNA molecules were blunt-ended in 200µl containing 50mM Tris pH 8.3, 10mM MgCl2, 10mM 2-mercaptoethanol, 50mM NaCl, 50µM of each deoxynucleotide triphosphate, 100ug/ml ovalbumin and 5 units of T4 polymerase. The reaction was incubated at 37° for 30 minutes and then terminated by phenol-chloroform extraction. Nucleic acids were then precipitated by ethanol addition.

EcoRl "linkers" (Collabortive Research) were then ligated to the blunted cDNA molecules under standard conditions, Manitis, et al., supra, at 243, in a total volume of 45ul. The reactions were terminated by the addition of EDTA to 15mM and extracted with phenol-chloroform. Nucleic acids were precipitated by ethanol and pelleted by centrifugation. The linkered cDNA was redissolved in 200ul of 100uM Tris-HCl (pH 7.2), 5mM MgCl2, 50mM NaCl and digested with 300 units of EcoRl for 2 hours at 37°C. The reaction was then extracted with phenol-chloroform and chromatographed over Sepharose CL-4B equilibrated with 10mM Tris (pH 8.0), 1mM EDTA, 0.1M NaCl. cDNA in the void volume was collected, precipitated by ethanol and pelleted by centrifugation.

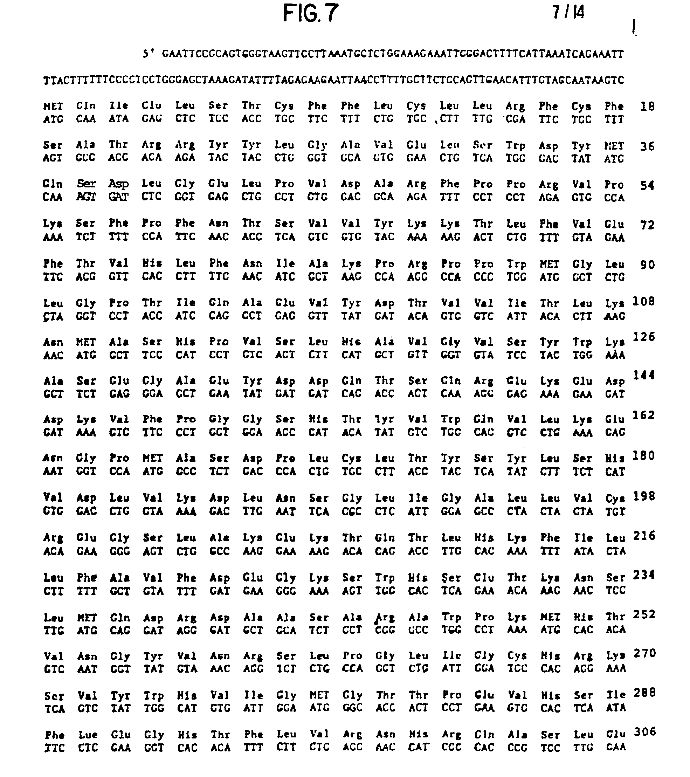

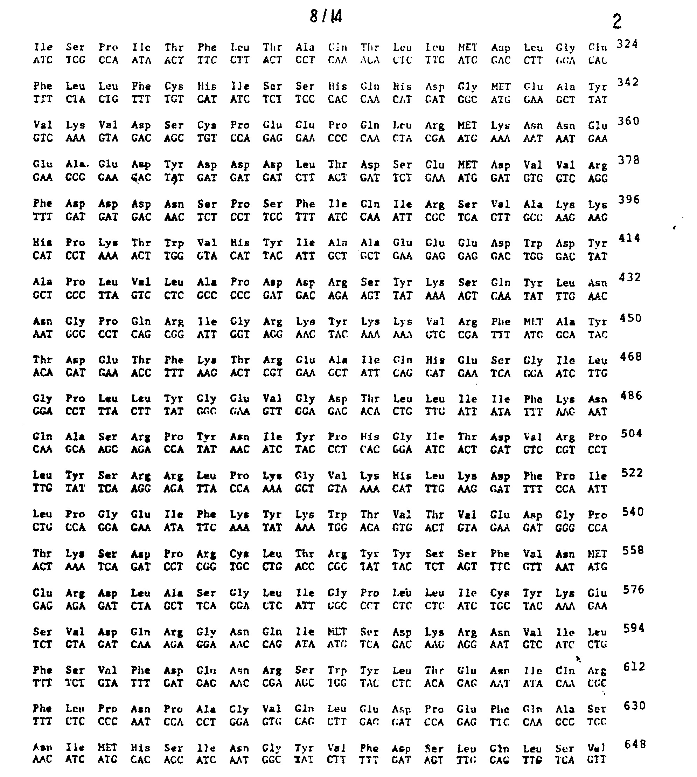

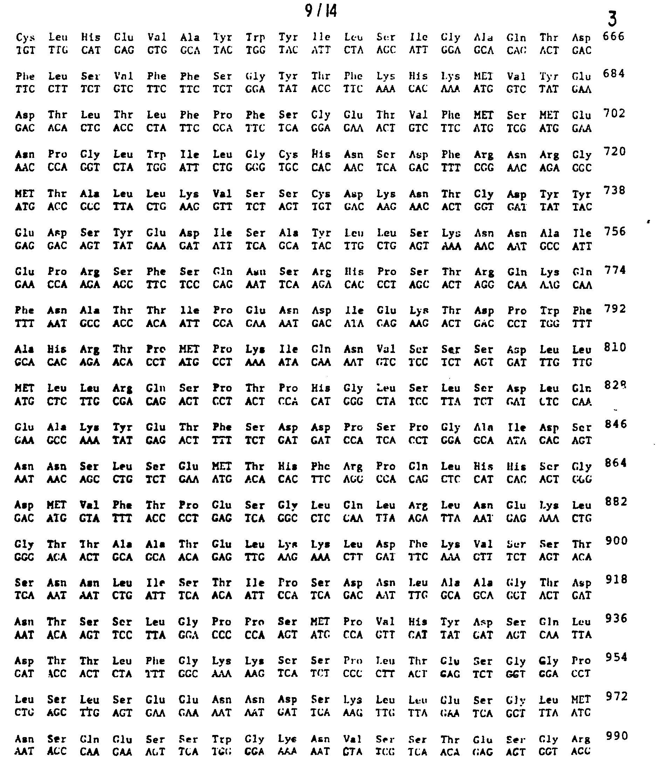

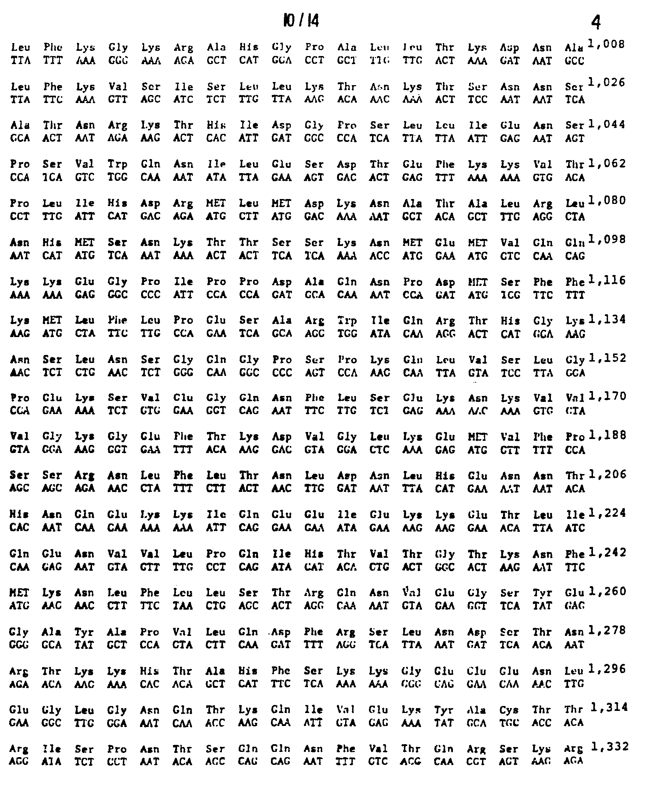

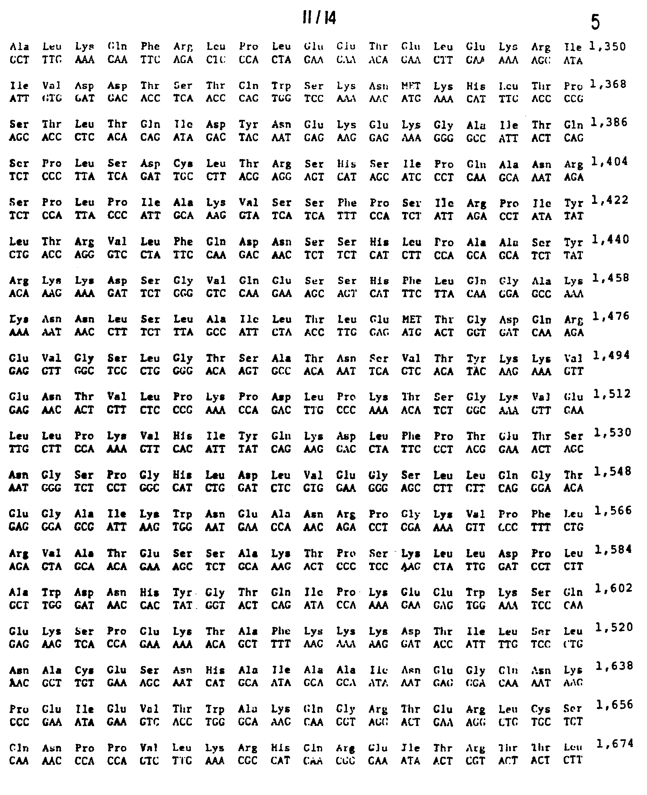

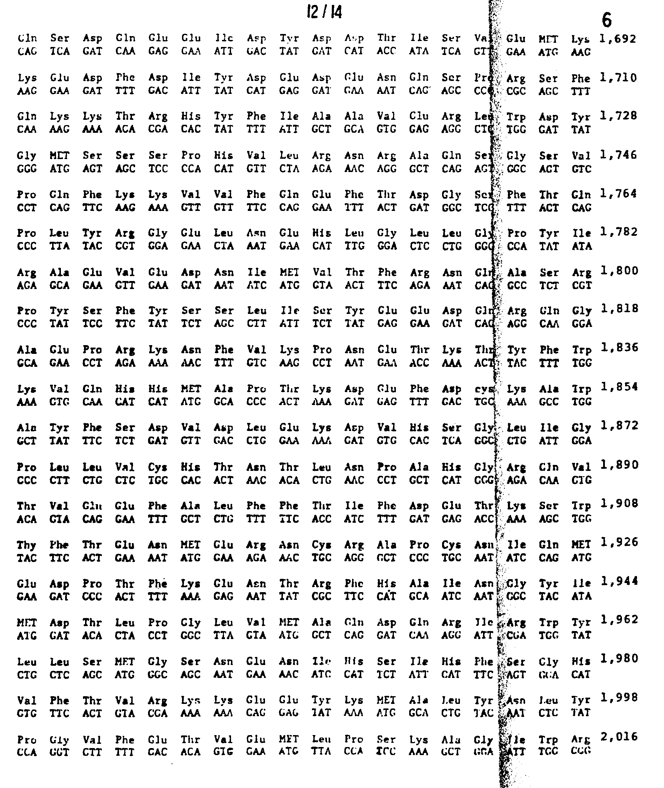

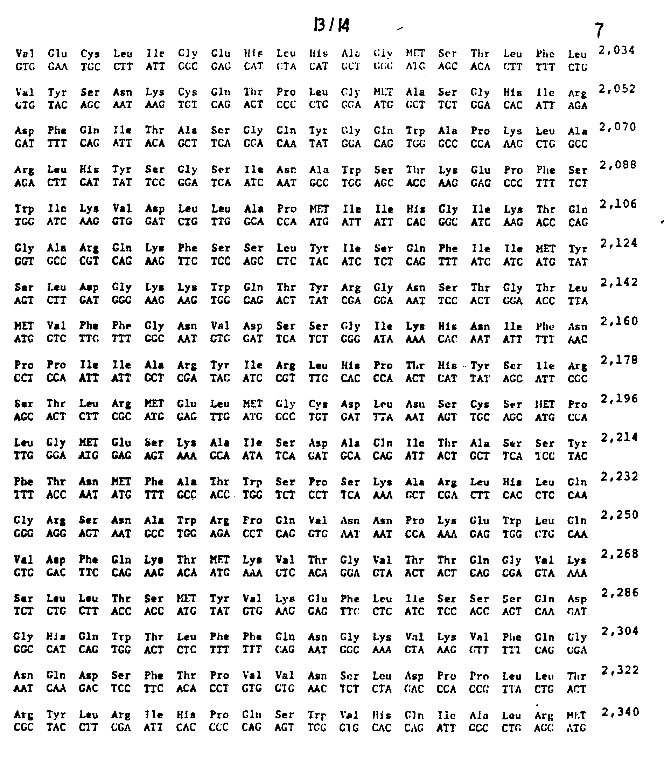

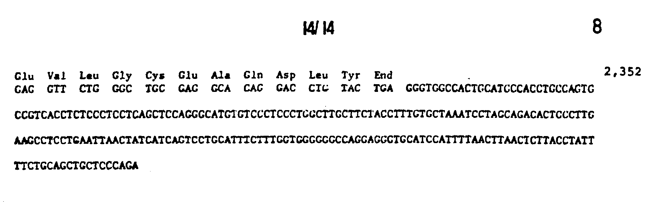

cDNA was redissolved in 10mM Tris-HCl (pH 8.0), 1mM EDTA and ligated to EcoRl cleaved, phosphatased lambda Charon 21A DNA at various ratios of cDNA to vector DNA using standard ligation conditions. Ligated DNA was packaged and titered using established procedures, Manitis, et al., supra, at 64, 256. The library was plated and screened using 32P-labelled human exon DNA under conditions described in Benton and Davis, supra. Overlapping clones which spanned approximately 10,000 base pairs were obtained and a substantial portion thereof was sequenced to reveal one long open reading frame encoding human AHF. The recombinant DNA nucleotide sequence obtained therefrom coding for human AHF is shown in Fig. 7 along with the deduced amino acid sequence for human AHF. The overlapping clones were assembled into vector pSP64 (Promega Biotec) using conventional techniques well known in the art, i.e. by ligation of DNA fragments from overlaping clones at resttriction enzyme sites common to both clones. A pSP64 recombinant clone containing the nucleotide sequence depicted in Figure 7, designated as pSP64-VIII, is on deposit at the American Type Culture Collection under Accession Number ATCC 39812.

## EXAMPLE 7
### Expression of human or porcine AHF

This example contemplates expression of AHF using the full length clone obtained by the method of Example 6 in a cotransformation system.

### 1. Preparation of Transformation Vector

A direct method for obtaining the AHF transformation vector is described below. The pCVSVL plasmid is partially digested with Pst I, the digest separated on gel electrophoresis. After visualization, the linear DNA fragment band corresponding to full length plasmid is isolated as pCVSVL-Bl.

The cDNA for AHF is rescued from the Example 5 cloning vectors by partial digest with Pst I. The partial digest is separated on gel electrophoresis, and the band corresponding to the molecular weight of the full length cDNA is isolated. pCVSVL-Bl is annealed with this DNA fragment, ligated with T4 ligase at 15°C, transfected into E. coli strain HB101 and transformants selected for tetracycline resistance. The selected E. coli transformants are grown in the presence of tetracycline. Plasmid pCVSVL-Bla is recovered in conventional fashion. Proper orientation of the cDNA in the plasmid may be determined in conventional fashion by assymetric digestion with an appropriate endonuclease(s).

### 2. Cotransformation: Selection and Amplification

Plasmid pCVSVL-Ala or pCVSVL-Bla and pAdD26SVpA#3 (Kaufman et al., op. cit.) are mixed together (50 $\mu$g HP and .5 $\mu$g pAdD265VpA #3) and precipitated by the addition of NaOAc pH 4.5 to .3M and 2.5 vols. of ethanol. Precipitated DNA is allowed to air dry, is resuspended in 2X HEBSS (.5ml) and mixed vigorously with .25 $M$ CaCl$_2$ (.5ml) as described (Kaufman et al., op. cit). The calcium-phosphate-DNA precipitate is allowed to sit 30' at room temperature and applied to CHO DUKX-B1 cells (Chasin and Urlaub, 1980, available from Columbia University). The growth and maintenance of these cells has been described (Kaufman et al., op. cit, Chasin and Urlaub 1980).

The DUKX-B1 cells are subcultured at 5x10$^5$/10cm dish 24 hr. prior to transfection. After 30 minutes incubation at room temperature, 5ml of a media with 10% fetal calf serum is applied and the cells are incubated at 37° for 4.5 hr. The media is then removed from the monolayer, 2ml of a-media with 10% fetal calf serum, 10$\mu$g/ml of thymidine, adenosine, and deoxyadenosine, and penicillin and streptomycin. Two days later the cells are subcultured 1:15 into a-media with 10% dialyzed fetal calf serum, and penicillin and streptomycin, but lacking nucleosides. Cells are then fed again with the same media after 4-5 days.

Colonies appear 10-12 days after subculturing into selective media. Methotrexate (MTX) selection and detection of AHF gene and selection gene amplification are conducted in accordance with Axel et a., U.S. Patent 4,399,216, or Kaufman et al., op. cit.

AHF yields may be improved by cotransforming the permanent cell line EA.hy 926 (Edgell et al., "Proc. Natl. Acad. Sci" 80:3734-3737 (1983) in place of DUKX-Bl cells. In this case the cotransforming selection gene should be the dominant DHFR gene disclosed by Haber et al., "Somatic Cell Genetics" 4:499-508 (1982). Otherwise, the cotransformation and culture will be substantially as set forth elsewhere herein.

### 3. Production of AHF

AHF-producing CHO transformants selected in Section 2 are maintained in seed culture using standard techniques. The culture is scaled up to 10 liters by cell culture in conventional media. This medium need not contain MTX and will not contain nucleosides so as to exclude selection gene revertants.

The culture supernatant is monitored for clotting activity following standard assays for use with blood plasma samples (reduction in clotting time of Factor VIII-deficient plasma). The supernatant may be purified by conventional techniques such as polyethylene glycol and glycine precipitations (as are known for use in purifying AHF from blood plasma) in order to increase the sensitivity of the FACTOR VIII assays.

When FACTOR VIII activity has reached a peak the cells are separated from the culture medium by centrifugation. The Factor VIII is then recovered and purified by polyethylene glycol and glycine precipitations. Clotting activity is demonstrated in Factor VIII deficient plasma.

### 4. Alternative Procedure for Production of AHF

A full length cDNA containing the entire AHF coding region was constructed from the exon in HH-25 described above, two cDNA clones which overlapped the extreme 5' and 3' regions of that exon, and a third clone which overlapped the 3' cDNA clone and continued past the termination codon. This was constructed such that synthetic Sal I sites were placed just 5' to the initiator methionine and at a position 3' to the translational stop signal at the end of the AHF coding sequences. This allowed

0182448

the placement into and excision from the polylinker Sal I site of pSP64. The Sal I fragment from this clone (pSP64-VIII) was purified and ligated to pCVSVL2. pCVSVL2 is a plasmid which is identical to the expression vector pCVSVL (Kaufman, et al., Mol. Cell Biol., 2:1304 (1982) the disclosure of which in incorporated herein by reference), except that it has deleted the Pst I site located 3' of the SV40 polyadenylation sequence which is accomplished by conventional procedures and that it contains a duplication of the SV40 Ava II "D" fragment upstream from the adenovirus major late promoter (MLP). pCVSVL2 was derived from pAdD26SVpA(1) (See Kaufman et al., supra) by adding XhoI linkers at each end of two SV40 Ava II "D" fragments, and inserting them into the XhoI site in pAdD26SVpA(1). The Ava II "D" fragments are both inserted such that the SV40 late promoter is in the same orientation as the Ad2 major late promoter. For plasmids pCVSVL and pAdD26SVpA(3), see Kaufman et. al., supra. To insert the Sal I fragment of pSP64-VIII into pCVSVL2, the unique Pst I site in pCVSVL2 was converted to a Sal I site by the procedure described in Rothstein et al., Methods Enzym., 69:98 (1980), and the Sal I fragment excised from pSP64-VIII was inserted to yield pCVSVL2-VIII. pCVSVL2-VIII contains the correct 5' to 3' orientation which allows transcription of the AHF coding sequence from the adenovirus MLP of the vector. pCVSVL2-VIII is on deposit at the American Type Culture Collection under Accession Number _39813_ .

The pCVSVL2-VIII was introduced into monkey COS-7 cells (Mellon et al., Cell 27:279 (1981) using the DEAE dextran transfection protocol described by Sampayrac and Danna, PNAS U.S.A. 78:7575 (1981). The cells were cultured in serum-free medium which was assayed for AHF activity 3 days after transfection.

Factor VIII:C activity was determined by the chromogenic substrate assay, described by Didisheim, Science 129:389 (1959), which confirmed that human Factor VIII:C expression had been

0182448

achieved at a level of about 0.05 units/ml.

CLAIMS

1. A DNA sequence coding for human factor VIII:C substantially free of other human genes.

2. The DNA sequence of Claim 1, wherein said sequence comprises one or more DNA sequences selected from the DNA nucleotide sequence depicted in Figure 7 or one or more DNA sequences which hybridize to the DNA nucleotide sequence depicted in Figure 7 and which on expression codes for a polypeptide which exhibits factor VIII:C activity.

3. The DNA sequence of Claim 1, wherein said sequence comprises or codes for a polypeptide having the amino sequence shown in Figure 7.

4. A transformed host containing a gene for human factor VIII:C, said host being selected from bacteria, yeasts, and mammalian cells.

5. Isolated DNA coding for human factor VIII:C, comprising a polydeoxyribonucleotide having the sequence:

5'OGC AGC TTT CAG AAG AAA ACA CGA CAC TAT TTT ATT GCT GCA GTG GAG AGG 3'

6. The DNA of Claim 5, excised from human genomic DNA.

7. The DNA of Claim 5, linked directly or indirectly to DNA from a non-human source.

8. An expression vector for human factor VIII:C, comprising the DNA segment of Claim 5.

9. A screening agent for identifying deoxyribonucleotide sequences and ribonucleotide sequences which encode for at least a portion of the human gene factor VIII:C, comprising a deoxyribonucleotide having at least a ten nucleotide portion of the sequence or its inverse complement:

5'TTT CAG AAG AGA ACC CGA CAC TAT TTC

  ATT GCT GCG GTG GAG CAG CTC TGG GAT

  TAC GGC ATG AGC GAA TCC CCC CGG GCG

  CTA AGA AAC AGG 3'.

10. A method of isolating a gene fragment with encodes at least a portion of protein, comprising

(a) forming a genomic DNA library of an organism that produces said protein,

(b) forming at least one pool of oligonucleotide probes whose sequence was selected solely based on the amino acid sequence contained in said protein and comprising a plurality of oligonucleotides which contain differing DNA sequences, each of which corresponds in DNA sequence to said amino acid sequence,

(c) contacting the genomic library with said pool under conditions favoring DNA/DNA hybridization,

(d) identifying the genomic DNA which hybridizes to the oligonucleotide in said pool, and

(e) isolating a segment of said genomic DNA which contains the oligonucleotide-hyridizing DNA.

11. The method according to Claim 10, wherein at least on oligonucleotide pool contains a plurality of oligonucleotides, one of which contains each possible DNA sequence which corresponds with a selected amino acid sequence in the protein.

12. The method of Claim 10, wherein the oligonucleotides have a length of from about 10 to 200 nucleotides and preferably 10 to 20 nucleotides.

13. The method of Claim 10, wherein the oligonucleotides have a chain length of from about 30 to 200 nucleotides.

14. A method in accordance with Claim 10, wherein at least two oligonucleotide probes are formed corresponding in DNA sequence to amino acid sequences in the protein, and each oligonucleotide probe is contacted with the genomic DNA under hybridizing conditions.

15. The method of Claim 14, wherein one oligonucleotide probe comprises a plurality of oligonucleotides having a length of 11 to 20 nucleotides, and another oligonucleotide probe comprises at least one oligonucleotide having a length of 40 to 200 nucleotides.

16. A method of isolating a gene which encodes for human factor VIII:C, comprising screening a cRNA library with a probe which corresponds to at least a ten nucleotide sequence of the following sequence or its inverse complement:

5'CGC AGC TTT CAA AAG AAA ACA CGA CAC TAT TTT ATT GCT GCA GTG GAG AGG 3'

constructing a cDNA library from the mRNA which hybridizes with the probe, and forming a gene which encodes for human factor VIII:C by ligating AHF cDNA segments from the cDNA library.

17. A method for producing AHF, comprising

(a) preparing one or more oligonucleotide probes which hybridize with human DNA which encodes AHF, or which hybridize with the complement of such DNA;

(b) using at least one such probe to identify cells containing mRNA transcripts having nucleotide sequences corresponding to AHF;

(c) preparing cDNA from the step (b) mRNA;

(d) assembling the step (c) cDNA into a replicable cDNA sequence encoding at least the amino acid sequence of mature human AHF;

(e) cloning the step (d) sequence;

(f) transforming a parental cell which does not express AHF with the step (e) sequence;

(g) culturing the step (f) transformed cell; and

(h) recovering AHF from the culture.

18. The method of Claim 17 wherein the cell is a mammalian cell.

19. A recombinant DNA sequence which codes for a polypeptide which produces factor VIII:C activity.

20. The DNA sequence of Claim 19, wherein the polypeptide comprises at least a major portion of the amino acid sequence of Figure 7.

21. A polypeptide expressed by DNA which exhibits human factor VIII:C activity comprising amino acid sequences selected from the amino acid sequence depicted in Figure 7 or amino acid sequences expressed by DNA sequences which hybridize to the DNA nucleotide sequence depicted in Figure 7.

22. The polypeptide of Claim 21, wherein said polypeptide comprises amino acids corresponding to a segment of the amino acid sequence of Figure 7 sufficient to provide factor VIII:C activity, substantially free of human fibrinogen and fibronectin.

23. The polypeptide of Claim 21, wherein said polypeptide is substantially free from other human polypeptides.

24. The polypeptide of Claim 23, wherein said other human polypeptides are human factor VIII:vWF, fibrinogen and fibronectin.

25. A pharmaceutical preparation useful for therapeutic treatment of Hemophilia A comprising a sterile preparation of the polypeptide of any one of Claims 21 to 24.

# FIG. I

A. Amino Acid Sequence of 69 Kd Porcine AHF Fragment

| | Phe | Gln | Lys | Arg | Thr | Arg | His | Tyr | Phe |
|---|---|---|---|---|---|---|---|---|---|
| | Ile | Ala | Ala | Val | Glu | Gln | Leu | Trp | Asp |
| | Tyr | Gly | Met | Ser | Glu | Ser | Pro | Arg | Ala |
| | Leu | Arg | Asn | Arg | Ala | Gln | Asn | Gly | Glu |
| | Val | Pro | Arg | Phe | Lys | | | | |

B. Amino Acid Sequence Encoded by Porcine AHF Exon (34-H1)

| Arg | Ser | Phe | Gln | Lys | Arg | Thr | Arg | His | Tyr | Phe |
|---|---|---|---|---|---|---|---|---|---|---|
| | | Ile | Ala | Ala | Val | Glu | Gln | Leu | Trp | Asp |
| | | Tyr | Gly | Met | Ser | Glu | Ser | Pro | Arg | Ala |

C. Amino Acid Sequence Encoded in Human AHF Exon (25-S1)

| Arg | Ser | Phe | Gln | Lys | Lys | Thr | Arg | His | Tyr |
|---|---|---|---|---|---|---|---|---|---|
| | Phe | Ile | Ala | Ala | Val | Glu | Arg | | |

# FIG. 2

### A. Amines Terminus of 166Kd Polypeptide

X I R R Y Y L G A V E L S W D Y R Q S E L L R E L H V D T R F P A

### B. Fragment of 166Kd Polypeptide

X V A K K H P K T W V H Y I S A E E E D W D Y A P A V P S P S D R S/T Y K S L

## FIG.3

| Amino Acid No. | 8 | 9 | 10 | 11 | 12 | 13 | 14 | 15 | 16 | 17 | 18 | 19 | 20 | 21 | 22 |
|---|---|---|---|---|---|---|---|---|---|---|---|---|---|---|---|
| Amino Acid Sequence | His | Tyr | Phe | Ile | Ala | Ala | Val | Glu | Gln | Leu | Trp | Asp | Tyr | Gly | Met |
| Possible mRNA Sequences | CAU CAC | UAU UAC | UUU UUC | AUU AUC AUA | GCU GCC GCA GCG | GCU GCC GCA GCG | GUU GUC GUA GUG | GAA GAG | CAA CAG | CUU CUC CUA CUG UUA UUG | UGG | GAU GAC | UAU UAC | GGU GGC GGA GBG | AUG |
| Guessed Probe Sequence | GTG | ATG | AAA AAG | TAA | CGA | CGA | CAC | CTT CTC | GTC | GAC | ACC | CTA CTG | ATA | CCG | TAC |
| Actual Probe Sequence | GTG | ATA | AAG | TAA | CGA | CGC | CAC | CTC | GTC | GAG | ACC | CTA | ATG | CCC | TAC |

# FIG.4

| | | | | | | | | 27 |
|---|---|---|---|---|---|---|---|---|
| AAA | ACA | CGG | GGC | ACC | TGT | TAA | CCT | GAA |
| CAA | AGT | AAA | TAG | ACC | TGG | AAG | GAC | 54 TCC |
| CTC | CAA | GCT | TCT | GGG | TCC | CCC | GAT | 81 GCC |
| CAA | AGA | GTG | GGA | ATC | CCT | AGA | GAA | 108 GTC |
| ACC | AAA | AAG | CAA | AGC | TCT | CAG | GAC | 135 GAA |
| AGA | CAT | CAT | CAG | TTT | ACC | CCT | GGA | 162 CCG |
| TCA | CGA | AAG | CAA | TCA | TTC | AAT | AGC | 189 AGC |
| AAA | AAA | TGA | AGG | ACA | AGC | CGA | GAC | 216 CCA |
| AAG | AGA | AGC | CGC | CTG | GAC | GAA | GCA | 243 GGG |
| AGG | GCC | TGG | AAG | GCT | GTG | CGC | TCC | 270 AAA |
| GCC | TCC | GGT | CCT | GCG | ACG | GCA | TCA | 297 GAG |
| GGA | CAT | AAG | CCT | TCC | TAC | TTT | TJA* | 324 CCC |
| GGA | GGA | AGA | CAA | AAT | GGA | CTA | TGA | 351 TGA |
| TAT | CTT | CTA | ACT | GAA | ACG | AAG | GGA | 378 GAA |
| GAT | TTT | GAC | ATT | TAC | GGT | GAG | GAT | 405 GAA |
| AAT | CAG | GAC | CCT | CGC | AGC | TTT | CAG | 432 AAG |
| AGA | ACC | CGA | CAC | TAT | TTC | ATT | GCT | 459 GCG |
| GTG | GAG | CAG | CTC | TGG | GAT | TAC | GGG | 486 ATG |
| AGC | GAA | TCC | CCC | CGG | GCG | CTA | AGA | 513 AAC |
| AGG | TAT | GGC | TAC | GTT | GGC | TAC | TCC | 540 TCT |
| GTC | CTA | CCC | TGG | GGA | CCT | TTG | TCT | 567 TGA |
| GCA | GGT | GCC | GAA | GCC | ATG | GGA | AAG | 594 GCA |
| CAA | GCA | GTC | TGG | GGG | TGG | AGA | GGC | 621 CAC |
| AGT | GGG | AGG | ATG | TGC | TTG | TTG | GGG | 648 AGC |
| ACA | GCG | TGG | TCG | GGC | AGG | GAA | GAG | 675 CAG |
| ACC | GAC | CTG | AGG | AGA | G | | | |

\* J Indicates an ambiguity, in accordance with the Stanford code.

# FIG.5

(250)

5'    TGG   AAG   GCT   GTG   CGC   TCC   AAA   GCC   TCC   GGT   CCT
                 9                      18                     27

           36                      45                                      63
      GCG   ACG   GCA   TCA   GAG   GGA   CAT   AAG   CCT   TCC   TAC

                 72                      81                      90                     99
      TTT   TJA   CCC   GGA   GGA   AGA   CAA   AAT   GGA   CTA   TGA

                       108                      117                     126
      TGA   TAT   CTT   CTA   ACT   GAA   ACG   AAG   GGA   GAA   GAT

           135                      144                     153                     162
      TTT   GAC   ATT   TAC   GGT   GAG   GAT   GAA   AAT   CAG   GAC

                 171                      180                     189        .            198
      CCT   CGC   AGC   TTT   CAG   AAG   AGA   ACC   CGA   CAC   TAT
             R     S     F     Q     K     R     T     R     H     Y

                       207                      216                     225
      TTC   ATT   GCT   GCG   GTG   GAG   CAG   CTC   [TGG   GAT   TAC
       F     I     A     A     V     E     Q     L     W      D     Y

           234                      243                     252                     261
      GGG   ATG]  AGC   GAA   TCC   CCC   CGG   GCG   CTA   AGA
       G     M     S     E     S     P     R     A     L     R

                 267
      AAC   AGG   TAT   GGC   TAC   GTT   GGC   TAC   TCC   TCT
       N     R

      GTC   CTA   CCC   TGG   GGA   CCT   TTC   TCT   TGA   GCA

      GGT   GCC   GAA   GCC   ATG   GGA   AAG   GCA   CAA   GCA

      GTC   TGG   GGG   TGG   AGA   (3')

## FIG.6

<u>HUMAN SEQ. DATA</u>   (Complement of 15 mer desired sequence)

| 5' | GAC | ATT | TAT | GAT | GAG | GAT | GAA |
|----|-----|-----|-----|-----|-----|-----|-----|
|    | ATT | CAG | AGC | CCC | CGC | AGC | TTT |
|    | CAG | AAG | AAA | ACA | CGA | CAC | TAT |
|    | TTT | ATT | GCT | GCA | GTG | GAG | AGG |

5' GAATTCCGCAGTGGGTAAGTTCCTTAAATGCTCTGGAAAGAAATTCGGACTTTTCATTAAATCAGAAATT

TTACTTTTTTCCCCTCCTGGGAGCTAAAGATATTTTAGAGAAGAATTAACCTTTTGCTTCTCCACTTGAACATTTCTAGCAATAAGTC

| | | | | | | | | | | | | | | | | | |
|---|---|---|---|---|---|---|---|---|---|---|---|---|---|---|---|---|---|---|
| MET | Gln | Ile | Glu | Leu | Ser | Thr | Cys | Phe | Phe | Leu | Cys | Leu | Leu | Arg | Phe | Cys | Phe | 18 |
| ATG | CAA | ATA | GAG | CTC | TCC | ACC | TGC | TTC | TTT | CTG | TGC | CTT | TTG | CCA | TTC | TGC | TTT | |
| Ser | Ala | Thr | Arg | Arg | Tyr | Tyr | Leu | Gly | Ala | Val | Glu | Leu | Ser | Trp | Asp | Tyr | MET | 36 |
| AGT | GCC | ACC | AGA | AGA | TAC | TAC | CTC | GGT | CCA | GTG | GAA | CTG | TCA | TGG | GAC | TAT | ATC | |
| Gln | Ser | Asp | Leu | Gly | Glu | Leu | Pro | Val | Asp | Ala | Arg | Phe | Pro | Pro | Arg | Val | Pro | 54 |
| CAA | AGT | GAT | CTC | GGT | GAG | CTG | CCT | CTC | GAC | GCA | AGA | TTT | CCT | CCT | AGA | GTG | CCA | |
| Lys | Ser | Phe | Pro | Phe | Asn | Thr | Ser | Val | Val | Tyr | Lys | Lys | Thr | Leu | Phe | Val | Glu | 72 |
| AAA | TCT | TTT | CCA | TTC | AAC | ACC | TCA | GTC | GTG | TAC | AAA | AAG | ACT | CTG | TTT | GTA | GAA | |
| Phe | Thr | Val | His | Leu | Phe | Asn | Ile | Ala | Lys | Pro | Arg | Pro | Pro | Trp | MET | Gly | Leu | 90 |
| TTC | ACG | GTT | CAC | CTT | TTC | AAC | ATC | GCT | AAG | CCA | AGG | CCA | CCC | TGG | ATG | GCT | CTG | |
| Leu | Gly | Pro | Thr | Ile | Gln | Ala | Glu | Val | Tyr | Asp | Thr | Val | Val | Ile | Thr | Leu | Lys | 108 |
| CTA | GGT | CCT | ACC | ATC | CAG | GCT | GAG | GTT | TAT | GAT | ACA | GTG | GTC | ATT | ACA | CTT | AAG | |
| Asn | MET | Ala | Ser | His | Pro | Val | Ser | Leu | His | Ala | Val | Gly | Val | Ser | Tyr | Trp | Lys | 126 |
| AAC | ATG | GCT | TCC | CAT | CCT | GTC | ACT | CTT | CAT | GCT | GTT | GGT | GTA | TCC | TAC | TGG | AAA | |
| Ala | Ser | Glu | Gly | Ala | Glu | Tyr | Asp | Asp | Gln | Thr | Ser | Gln | Arg | Glu | Lys | Glu | Asp | 144 |
| GCT | TCT | GAG | GGA | GCT | GAA | TAT | GAT | GAT | CAG | ACC | ACT | CAA | AGC | GAG | AAA | GAA | GAT | |
| Asp | Lys | Val | Phe | Pro | Gly | Gly | Ser | His | Thr | Tyr | Val | Trp | Gln | Val | Leu | Lys | Glu | 162 |
| GAT | AAA | GTC | TTC | CCT | GGT | GGA | AGC | CAT | ACA | TAT | GTC | TGG | CAG | GTC | CTG | AAA | GAG | |
| Asn | Gly | Pro | MET | Ala | Ser | Asp | Pro | Leu | Cys | Leu | Thr | Tyr | Ser | Tyr | Leu | Ser | His | 180 |
| AAT | GGT | CCA | ATG | GCC | TCT | GAC | CCA | CTG | TGC | CTT | ACC | TAC | TCA | TAT | CTT | TCT | CAT | |
| Val | Asp | Leu | Val | Lys | Asp | Leu | Asn | Ser | Gly | Leu | Ile | Gly | Ala | Leu | Leu | Val | Cys | 198 |
| GTC | GAC | CTG | GTA | AAA | GAC | TTC | AAT | TCA | GGC | CTC | ATT | GGA | GCC | CTA | CTA | CTA | TGT | |
| Arg | Glu | Gly | Ser | Leu | Ala | Lys | Glu | Lys | Thr | Gln | Thr | Leu | His | Lys | Phe | Ile | Leu | 216 |
| ACA | GAA | GGG | AGT | CTG | GCC | AAG | GAA | AAG | ACA | CAC | ACC | TTG | CAC | AAA | TTT | ATA | CTA | |
| Leu | Phe | Ala | Val | Phe | Asp | Glu | Gly | Lys | Ser | Trp | His | Ser | Glu | Thr | Lys | Asn | Ser | 234 |
| CTT | TTT | GCT | GTA | TTT | GAT | GAA | GGG | AAA | AGT | TGG | CAC | TCA | GAA | ACA | AAG | AAC | TCC | |
| Leu | MET | Gln | Asp | Arg | Asp | Ala | Ala | Ser | Ala | Arg | Ala | Trp | Pro | Lys | MET | His | Thr | 252 |
| TTC | ATG | CAG | GAT | AGG | GAT | GCT | GCA | TCT | GCT | CGG | GCC | TGG | CCT | AAA | ATG | CAC | ACA | |
| Val | Asn | Gly | Tyr | Val | Asn | Arg | Ser | Leu | Pro | Gly | Leu | Ile | Gly | Cys | His | Arg | Lys | 270 |
| GTC | AAT | GGT | TAT | GTA | AAC | AGG | TCT | CTG | CCA | GGT | CTG | ATT | GGA | TCC | CAC | AGG | AAA | |
| Ser | Val | Tyr | Trp | His | Val | Ile | Gly | MET | Gly | Thr | Thr | Pro | Glu | Val | His | Ser | Ile | 288 |
| TCA | GTC | TAT | TGG | CAT | GTG | ATT | GGA | ATG | GGC | ACC | ACT | CCT | GAA | GTC | CAC | TCA | ATA | |
| Phe | Lue | Glu | Gly | His | Thr | Phe | Leu | Val | Arg | Asn | His | Arg | Gln | Ala | Ser | Leu | Glu | 306 |
| TTC | CTC | GAA | GGT | CAC | ACA | TTT | CTT | CTC | AGG | AAC | CAT | CGC | CAC | CCG | TCC | TTG | GAA | |

0182448

2

```
Ile Ser Pro Ile Thr Phe Leu Thr Ala Gln Thr Leu Leu MET Asp Leu Gly Gln  324
ATC TCG CCA ATA ACT TTC CTT ACT GCT CAA ACA CTC TTG ATG GAC CTT GGA CAC

Phe Leu Leu Phe Cys His Ile Ser Ser His Gln His Asp Gly MET Glu Ala Tyr  342
TTT CTA CTG TTT TGT CAT ATC TCT TCC CAC CAA CAT GAT GGC ATG GAA GCT TAT

Val Lys Val Asp Ser Cys Pro Glu Glu Pro Gln Leu Arg MET Lys Asn Asn Glu  360
GTC AAA GTA GAC AGC TGT CCA GAG GAA CCC CAA CTA CGA ATG AAA AAT AAT GAA

Glu Ala Glu Asp Tyr Asp Asp Asp Leu Thr Asp Ser Glu MET Asp Val Val Arg  378
GAA GCG GAA GAC TAT GAT GAT GAT CTT ACT GAT TCT GAA ATG GAT GTG GTC AGG

Phe Asp Asp Asp Asn Ser Pro Ser Phe Ile Gln Ile Arg Ser Val Ala Lys Lys  396
TTT GAT GAT GAC AAC TCT CCT TCC TTT ATC CAA ATT CGC TCA GTT GCC AAG AAG

His Pro Lys Thr Trp Val His Tyr Ile Ala Ala Glu Glu Glu Asp Trp Asp Tyr  414
CAT CCT AAA ACT TGG GTA CAT TAC ATT GCT GCT GAA GAG GAG GAC TGG GAC TAT

Ala Pro Leu Val Leu Ala Pro Asp Asp Arg Ser Tyr Lys Ser Gln Tyr Leu Asn  432
GCT CCC TTA GTC CTC GCC CCC GAT GAC AGA AGT TAT AAA AGT CAA TAT TTG AAC

Asn Gly Pro Gln Arg Ile Gly Arg Lys Tyr Lys Lys Val Arg Phe MET Ala Tyr  450
AAT GGC CCT CAG CGG ATT GGT AGG AAC TAC AAA AAA GTC CGA TTT ATG GCA TAC

Thr Asp Glu Thr Phe Lys Thr Arg Glu Ala Ile Gln His Glu Ser Gly Ile Leu  468
ACA GAT GAA ACC TTT AAG ACT CGT GAA GCT ATT CAG CAT GAA TCA GGA ATC TTG

Gly Pro Leu Leu Tyr Gly Glu Val Gly Asp Thr Leu Leu Ile Ile Phe Lys Asn  486
GGA CCT TTA CTT TAT GGG GAA GTT GGA GAC ACA CTG TTG ATT ATA TTT AAC AAT

Gln Ala Ser Arg Pro Tyr Asn Ile Tyr Pro His Gly Ile Thr Asp Val Arg Pro  504
CAA GCA AGC AGA CCA TAT AAC ATC TAC CCT CAC GGA ATC ACT GAT GTC CGT CCT

Leu Tyr Ser Arg Arg Leu Pro Lys Gly Val Lys His Leu Lys Asp Phe Pro Ile  522
TTG TAT TCA AGG ACA TTA CCA AAA GGT GTA AAA CAT TTG AAG GAT TTT CCA ATT

Leu Pro Gly Glu Ile Phe Lys Tyr Lys Trp Thr Val Thr Val Glu Asp Gly Pro  540
CTG CCA GGA GAA ATA TTC AAA TAT AAA TGG ACA GTG ACT GTA GAA GAT GGG CCA

Thr Lys Ser Asp Pro Arg Cys Leu Thr Arg Tyr Tyr Ser Ser Phe Val Asn MET  558
ACT AAA TCA GAT CCT CGG TGC CTG ACC CGC TAT TAC TCT AGT TTC GTT AAT ATG

Glu Arg Asp Leu Ala Ser Gly Leu Ile Gly Pro Leu Leu Ile Cys Tyr Lys Glu  576
GAG AGA GAT CTA GCT TCA GGA CTC ATT GGC CCT CTC CTC ATC TGC TAC AAA GAA

Ser Val Asp Gln Arg Gly Asn Gln Ile MET Ser Asp Lys Arg Asn Val Ile Leu  594
TCT GTA GAT CAA AGA GGA AAC CAG ATA ATG TCA GAC AAG AGG AAT GTC ATC CTG

Phe Ser Val Phe Asp Glu Asn Arg Ser Trp Tyr Leu Thr Glu Asn Ile Gln Arg  612
TTT TCT GTA TTT GAT GAG AAC CGA AGC TGG TAC CTC ACA CAG AAT ATA CAA CGC

Phe Leu Pro Asn Pro Ala Gly Val Gln Leu Glu Asp Pro Glu Phe Gln Ala Ser  630
TTT CTC CCC AAT CCA CCT GGA GTG CAG CTT GAG GAT CCA GAG TTC CAA GCC TCC

Asn Ile MET His Ser Ile Asn Gly Tyr Val Phe Asp Ser Leu Gln Leu Ser Val  648
AAC ATC ATG CAC AGC ATC AAT GGC TAT CTT TTT GAT AGT TTG CAG TTG TCA GTT
```

0182448

3

| Cys | Leu | His | Glu | Val | Ala | Tyr | Trp | Tyr | Ile | Leu | Ser | Ile | Gly | Ala | Gln | Thr | Asp | 666 |
|-----|-----|-----|-----|-----|-----|-----|-----|-----|-----|-----|-----|-----|-----|-----|-----|-----|-----|-----|
| TGT | TTG | CAT | GAG | CTG | GCA | TAC | TGG | TAC | ATT | CTA | AGC | ATT | GGA | GCA | CAC | ACT | GAC | |

| Phe | Leu | Ser | Val | Phe | Phe | Ser | Gly | Tyr | Thr | Phe | Lys | His | Lys | MET | Val | Tyr | Glu | 684 |
| TTC | CTT | TCT | GTC | TTC | TTC | TCT | GGA | TAT | ACC | TTC | AAA | CAC | AAA | ATG | GTC | TAT | GAA | |

| Asp | Thr | Leu | Thr | Leu | Phe | Pro | Phe | Ser | Gly | Glu | Thr | Val | Phe | MET | Ser | MET | Glu | 702 |
| GAC | ACA | CTC | ACC | CTA | TTC | CCA | TTC | TCA | GGA | GAA | ACT | GTC | TTC | ATG | TCG | ATG | GAA | |

| Asn | Pro | Gly | Leu | Trp | Ile | Leu | Gly | Cys | His | Asn | Ser | Asp | Phe | Arg | Asn | Arg | Gly | 720 |
| AAC | CCA | GGT | CTA | TGG | ATT | CTG | GGG | TGC | CAC | AAC | TCA | GAC | TTT | CGG | AAC | AGA | GGC | |

| MET | Thr | Ala | Leu | Leu | Lys | Val | Ser | Ser | Cys | Asp | Lys | Asn | Thr | Gly | Asp | Tyr | Tyr | 738 |
| ATG | ACC | GCC | TTA | CTG | AAG | GTT | TCT | AGT | TGT | GAC | AAG | AAC | ACT | GGT | GAT | TAT | TAC | |

| Glu | Asp | Ser | Tyr | Glu | Asp | Ile | Ser | Ala | Tyr | Leu | Leu | Ser | Lys | Asn | Asn | Ala | Ile | 756 |
| GAG | GAC | AGT | TAT | GAA | GAT | ATT | TCA | GCA | TAC | TTG | CTG | AGT | AAA | AAC | AAT | GCC | ATT | |

| Glu | Pro | Arg | Ser | Phe | Ser | Gln | Asn | Ser | Arg | His | Pro | Ser | Thr | Arg | Gln | Lys | Gln | 774 |
| GAA | CCA | AGA | ACC | TTC | TCC | CAG | AAT | TCA | AGA | CAC | CCT | AGC | ACT | AGG | CAA | AAG | CAA | |

| Phe | Asn | Ala | Thr | Thr | Ile | Pro | Glu | Asn | Asp | Ile | Glu | Lys | Thr | Asp | Pro | Trp | Phe | 792 |
| TTT | AAT | GCC | ACC | ACA | ATT | CCA | GAA | AAT | GAC | ATA | GAG | AAG | ACT | GAC | CCT | TGG | TTT | |

| Ala | His | Arg | Thr | Pro | MET | Pro | Lys | Ile | Gln | Asn | Val | Ser | Ser | Ser | Asp | Leu | Leu | 810 |
| GCA | CAC | AGA | ACA | CCT | ATG | CCT | AAA | ATA | CAA | AAT | GTC | TCC | ICT | AGT | GAT | TTG | TTG | |

| MET | Leu | Leu | Arg | Gln | Ser | Pro | Thr | Pro | His | Gly | Leu | Ser | Leu | Ser | Asp | Leu | Gln | 828 |
| ATG | CTC | TTC | CGA | CAG | ACT | CCT | ACT | CCA | CAT | GGG | CTA | TCC | TTA | TCT | GAT | CTC | CAA | |

| Glu | Ala | Lys | Tyr | Glu | Thr | Phe | Ser | Asp | Asp | Pro | Ser | Pro | Gly | Ala | Ile | Asp | Ser | 846 |
| GAA | GCC | AAA | TAT | GAG | ACT | TTT | TCT | GAT | GAT | CCA | TCA | CCT | GGA | GCA | ATA | CAC | AGT | |

| Asn | Asn | Ser | Leu | Ser | Glu | MET | Thr | His | Phe | Arg | Pro | Gln | Leu | His | His | Ser | Gly | 864 |
| AAT | AAC | AGC | CTG | TCT | GAA | ATG | ACA | CAC | TTC | AGC | CCA | CAG | CTC | CAT | CAC | AGT | GGG | |

| Asp | MET | Val | Phe | Thr | Pro | Glu | Ser | Gly | Leu | Gln | Leu | Arg | Leu | Asn | Glu | Lys | Leu | 882 |
| GAC | ATG | GTA | TTT | ACC | CCT | GAG | TCA | GGC | CTC | CAA | TTA | AGA | TTA | AAT | GAG | AAA | CTG | |

| Gly | Thr | Thr | Ala | Ala | Thr | Glu | Leu | Lys | Lys | Leu | Asp | Phe | Lys | Val | Ser | Ser | Thr | 900 |
| GGC | ACA | ACT | GCA | GCA | ACA | GAG | TTG | AAG | AAA | CTT | GAT | TTC | AAA | GTT | TCT | AGT | ACA | |

| Ser | Asn | Asn | Leu | Ile | Ser | Thr | Ile | Pro | Ser | Asp | Asn | Leu | Ala | Ala | Gly | Thr | Asp | 918 |
| TCA | AAT | AAT | CTG | ATT | TCA | ACA | ATT | CCA | TCA | GAC | AAT | TTG | GCA | GCA | GGT | ACT | GAT | |

| Asn | Thr | Ser | Ser | Leu | Gly | Pro | Pro | Ser | MET | Pro | Val | His | Tyr | Asp | Ser | Gln | Leu | 936 |
| AAT | ACA | AGT | TCC | TTA | GGA | CCC | CCA | AGT | ATG | CCA | GTT | CAT | TAT | GAT | AGT | CAA | TTA | |

| Asp | Thr | Thr | Leu | Phe | Gly | Lys | Lys | Ser | Ser | Pro | Leu | Thr | Glu | Ser | Gly | Gly | Pro | 954 |
| GAT | ACC | ACT | CTA | TTT | GGC | AAA | AAG | TCA | TCT | CCC | CTT | ACT | GAG | TCT | GGT | GGA | CCT | |

| Leu | Ser | Leu | Ser | Glu | Glu | Asn | Asn | Asp | Ser | Lys | Leu | Leu | Glu | Ser | Gly | Leu | MET | 972 |
| CTC | AGC | TTG | AGT | GAA | GAA | AAT | AAT | GAT | TCA | AAG | TTG | TTA | GAA | TCA | GGT | TTA | ATC | |

| Asn | Ser | Gln | Glu | Ser | Ser | Trp | Gly | Lys | Asn | Val | Ser | Ser | Thr | Glu | Ser | Gly | Arg | 990 |
| AAT | ACC | CAA | GAA | AGT | TCA | TGG | GGA | AAA | AAT | GTA | TCG | TCA | ACA | GAG | AGT | GGT | ACC | |

0182448

4

```
Leu  Phe  Lys  Gly  Lys  Arg  Ala  His  Gly  Pro  Ala  Leu  Leu  Thr  Lys  Asp  Asn  Ala 1,008
TTA  TTT  AAA  GGC  AAA  AGA  GCT  CAT  GGA  CCT  GCT  TTG  TTG  ACT  AAA  GAT  AAT  GCC

Leu  Phe  Lys  Val  Ser  Ile  Ser  Leu  Leu  Lys  Thr  Asn  Lys  Thr  Ser  Asn  Asn  Ser 1,026
TTA  TTC  AAA  GTT  AGC  ATC  TCT  TTG  TTA  AAG  ACA  AAC  AAA  ACT  TCC  AAT  AAT  TCA

Ala  Thr  Asn  Arg  Lys  Thr  His  Ile  Asp  Gly  Pro  Ser  Leu  Leu  Ile  Glu  Asn  Ser 1,044
GCA  ACT  AAT  AGA  AAG  ACT  CAC  ATT  GAT  GGC  CCA  TCA  TTA  TTA  ATT  GAG  AAT  AGT

Pro  Ser  Val  Trp  Gln  Asn  Ile  Leu  Glu  Ser  Asp  Thr  Glu  Phe  Lys  Lys  Val  Thr 1,062
CCA  TCA  GTC  TGG  CAA  AAT  ATA  TTA  GAA  AGT  GAC  ACT  GAG  TTT  AAA  AAA  GTG  ACA

Pro  Leu  Ile  His  Asp  Arg  MET  Leu  MET  Asp  Lys  Asn  Ala  Thr  Ala  Leu  Arg  Leu 1,080
CCT  TTG  ATT  CAT  GAC  AGA  ATG  CTT  ATG  GAC  AAA  AAT  GCT  ACA  GCT  TTG  AGG  CTA

Asn  His  MET  Ser  Asn  Lys  Thr  Thr  Ser  Ser  Lys  Asn  MET  Glu  MET  Val  Gln  Gln 1,098
AAT  CAT  ATG  TCA  AAT  AAA  ACT  ACT  TCA  TCA  AAA  ACC  ATG  GAA  ATG  GTC  CAA  CAG

Lys  Lys  Glu  Gly  Pro  Ile  Pro  Pro  Asp  Ala  Gln  Asn  Pro  Asp  MET  Ser  Phe  Phe 1,116
AAA  AAA  GAG  GGC  CCC  ATT  CCA  CCA  GAT  GCA  CAA  AAT  CCA  GAT  ATG  TCG  TTC  TTT

Lys  MET  Leu  Phe  Leu  Pro  Glu  Ser  Ala  Arg  Trp  Ile  Gln  Arg  Thr  His  Gly  Lys 1,134
AAG  ATG  CTA  TTC  TTG  CCA  GAA  TCA  GCA  AGG  TGG  ATA  CAA  AGG  ACT  CAT  GGA  AAG

Asn  Ser  Leu  Asn  Ser  Gly  Gln  Gly  Pro  Ser  Pro  Lys  Gln  Leu  Val  Ser  Leu  Gly 1,152
AAC  TCT  CTG  AAC  TCT  GGG  CAA  GGC  CCC  AGT  CCA  AAG  CAA  TTA  GTA  TCC  TTA  GGA

Pro  Glu  Lys  Ser  Val  Glu  Gly  Gln  Asn  Phe  Leu  Ser  Glu  Lys  Asn  Lys  Val  Val 1,170
CCA  GAA  AAA  TCT  GTG  GAA  GGT  CAG  AAT  TTC  TTG  TCT  GAG  AAA  AAC  AAA  GTG  CTA

Val  Gly  Lys  Gly  Glu  Phe  Thr  Lys  Asp  Val  Gly  Leu  Lys  Glu  MET  Val  Phe  Pro 1,188
GTA  GGA  AAG  GGT  GAA  TTT  ACA  AAG  GAC  GTA  GGA  CTC  AAA  GAG  ATG  CTT  TTT  CCA

Ser  Ser  Arg  Asn  Leu  Phe  Leu  Thr  Asn  Leu  Asp  Asn  Leu  His  Glu  Asn  Asn  Thr 1,206
AGC  AGC  AGA  AAC  CTA  TTT  CTT  ACT  AAC  TTG  GAT  AAT  TTA  CAT  GAA  AAT  AAT  ACA

His  Asn  Gln  Glu  Lys  Lys  Ile  Gln  Glu  Glu  Ile  Glu  Lys  Lys  Glu  Thr  Leu  Ile 1,224
CAC  AAT  CAA  CAA  AAA  AAA  ATT  CAG  GAA  GAA  ATA  GAA  AAG  AAG  GAA  ACA  TTA  ATC

Gln  Glu  Asn  Val  Val  Leu  Pro  Gln  Ile  His  Thr  Val  Thr  Gly  Thr  Lys  Asn  Phe 1,242
CAA  GAG  AAT  GTA  GTT  TTG  CCT  CAG  ATA  CAT  ACA  CTG  ACT  GGC  ACT  AAG  AAT  TTC

MET  Lys  Asn  Leu  Phe  Leu  Leu  Ser  Thr  Arg  Gln  Asn  Val  Glu  Gly  Ser  Tyr  Glu 1,260
ATG  AAC  AAC  CTT  TTC  TAA  CTG  ACC  ACT  AGG  CAA  AAT  GTA  GAA  GGT  TCA  TAT  GAG

Gly  Ala  Tyr  Ala  Pro  Val  Leu  Gln  Asp  Phe  Arg  Ser  Leu  Asn  Asp  Ser  Thr  Asn 1,278
GGG  GCA  TAT  GCT  CCA  GTA  CTT  CAA  GAT  TTT  AGG  TCA  TTA  AAT  GAT  TCA  ACA  AAT

Arg  Thr  Lys  Lys  His  Thr  Ala  His  Phe  Ser  Lys  Lys  Gly  Glu  Glu  Glu  Asn  Leu 1,296
AGA  ACA  AAC  AAA  CAC  ACA  GCT  CAT  TTC  TCA  AAA  AAA  GGC  GAG  GAA  CAA  AAC  TTG

Glu  Gly  Leu  Gly  Asn  Gln  Thr  Lys  Gln  Ile  Val  Glu  Lys  Tyr  Ala  Cys  Thr  Thr 1,314
GAA  GGC  TTG  GGA  AAT  CAA  ACC  AAG  CAA  ATT  GTA  GAG  AAA  TAT  GCA  TGC  ACC  ACA

Arg  Ile  Ser  Pro  Asn  Thr  Ser  Gln  Gln  Asn  Phe  Val  Thr  Gln  Arg  Ser  Lys  Arg 1,332
AGG  ATA  TCT  CCT  AAT  ACA  ACC  CAG  CAG  AAT  TTT  GTC  ACG  CAA  CGT  AGT  AAG  AGA
```

0182448

```
Ala Leu Lys Gln Phe Arg Leu Pro Leu Glu Glu Thr Glu Leu Glu Lys Arg Ile 1,350
GCT TTG AAA CAA TTC AGA CTC CCA CTA GAA CAA ACA GAA CAA CTT GAA AAA AGC ATA

Ile Val Asp Asp Thr Ser Thr Gln Trp Ser Lys Asn MET Lys His Leu Thr Pro 1,368
ATT GTG GAT GAC ACC TCA ACC CAG TGG TCC AAA AAC ATG AAA CAT TTC ACC CCG

Ser Thr Leu Thr Gln Ile Asp Tyr Asn Glu Lys Glu Lys Gly Ala Ile Thr Gln 1,386
AGC ACC CTC ACA CAG ATA GAC TAC AAT GAG AAG GAG AAA GGG GCC ATT ACT CAG

Ser Pro Leu Ser Asp Cys Leu Thr Arg Ser His Ser Ile Pro Gln Ala Asn Arg 1,404
TCT CCC TTA TCA GAT TGC CTT ACG AGG ACT CAT AGC ATC CCT CAA GCA AAT AGA

Ser Pro Leu Pro Ile Ala Lys Val Ser Ser Phe Pro Ser Ile Arg Pro Ile Tyr 1,422
TCT CCA TTA CCC ATT GCA AAG GTA TCA TCA TTT CCA TCT ATT AGA CCT ATA TAT

Leu Thr Arg Val Leu Phe Gln Asp Asn Ser Ser His Leu Pro Ala Ala Ser Tyr 1,440
CTG ACC AGG GTC CTA TTC CAA GAC AAC TCT TCT CAT CTT CCA GCA GCA TCT TAT

Arg Lys Lys Asp Ser Gly Val Gln Glu Ser Ser His Phe Leu Gln Gly Ala Lys 1,458
ACA AAG AAA GAT TCT GGG GTC CAA GAA AGC ACT CAT TTC TTA CAA GGA GCC AAA

Lys Asn Asn Leu Ser Leu Ala Ile Leu Thr Leu Glu MET Thr Gly Asp Gln Arg 1,476
AAA AAT AAC CTT TCT TTA GCC ATT CTA ACC TTG GAG ATG ACT GGT GAT CAA AGA

Glu Val Gly Ser Leu Gly Thr Ser Ala Thr Asn Ser Val Thr Tyr Lys Lys Val 1,494
GAG GTT GGC TCC CTG GGG ACA AGT GCC ACA AAT TCA CTC ACA TAC AAG AAA GTT

Glu Asn Thr Val Leu Pro Lys Pro Asp Leu Pro Lys Thr Ser Gly Lys Val Glu 1,512
GAG AAC ACT GTT CTC CCG AAA CCA GAC TTG CCC AAA ACA TCT GGC AAA GTT GAA

Leu Leu Pro Lys Val His Ile Tyr Gln Lys Asp Leu Phe Pro Thr Glu Thr Ser 1,530
TTG CTT CCA AAA GTT CAC ATT TAT CAG AAG GAC CTA TTC CCT ACG GAA ACT ACC

Asn Gly Ser Pro Gly His Leu Asp Leu Val Glu Gly Ser Leu Leu Gln Gly Thr 1,548
AAT GGG TCT CCT GGC CAT CTG GAT CTC GTG GAA GGG AGC CTT CTT CAG GGA ACA

Glu Gly Ala Ile Lys Trp Asn Glu Ala Asn Arg Pro Gly Lys Val Pro Phe Leu 1,566
GAG GGA GCG ATT AAG TGG AAT GAA CCA AAC AGA CCT CGA AAA GTT CCC TTT CTG

Arg Val Ala Thr Glu Ser Ser Ala Lys Thr Pro Ser Lys Leu Leu Asp Pro Leu 1,584
ACA GTA GCA ACA GAA AGC TCT GCA AAG ACT CCC TCC AAG CTA TTG GAT CCT CTT

Ala Trp Asp Asn His Tyr Gly Thr Gln Ile Pro Lys Glu Glu Trp Lys Ser Gln 1,602
GCT TGG GAT AAC CAC TAT GGT ACT CAG ATA CCA AAA GAA GAG TGG AAA TCC CAA

Glu Lys Ser Pro Glu Lys Thr Ala Phe Lys Lys Lys Asp Thr Ile Leu Ser Leu 1,520
GAG AAG TCA CCA GAA AAA ACA GCT TTT AAG AAA AAG GAT ACC ATT TTG TCC CTG

Asn Ala Cys Glu Ser Asn His Ala Ile Ala Ala Ile Asn Glu Gly Gln Asn Lys 1,638
AAC GCT TGT GAA AGC AAT CAT GCA ATA GCA GCA ATA AAT GAG GGA CAA AAT AAG

Pro Glu Ile Glu Val Thr Trp Ala Lys Gln Gly Arg Thr Glu Arg Leu Cys Ser 1,656
CCC GAA ATA GAA GTC ACC TGG GCA AAG CAA GGT AGG ACT GAA AGG CTC TGC TCT

Gln Asn Pro Pro Val Leu Lys Arg His Gln Arg Glu Ile Thr Arg Thr Thr Leu 1,674
CAA AAC CCA CCA GTC TTG AAA CGC CAT CAA CGG GAA ATA ACT CGT ACT ACT CTT
```

6

```
Gln  Ser  Asp  Gln  Glu  Glu  Ile  Asp  Tyr  Asp  Asp  Thr  Ile  Ser  Val  Glu  MET  Lys  1,692
CAG  TCA  GAT  CAA  GAG  GAA  ATT  GAC  TAT  GAT  CAT  ACC  ATA  TCA  GTT  GAA  ATG  AAG

Lys  Glu  Asp  Phe  Asp  Ile  Tyr  Asp  Glu  Asp  Glu  Asn  Gln  Ser  Pro  Arg  Ser  Phe  1,710
AAG  GAA  GAT  TTT  GAC  ATT  TAT  CAT  GAG  GAT  GAA  AAT  CAG  AGC  CCG  CGC  AGC  TTT

Gln  Lys  Lys  Thr  Arg  His  Tyr  Phe  Ile  Ala  Ala  Val  Glu  Arg  Leu  Trp  Asp  Tyr  1,728
CAA  AAG  AAA  ACA  CGA  CAC  TAT  TTT  ATT  GCT  GCA  GTG  GAG  AGG  CTC  TGG  GAT  TAT

Gly  MET  Ser  Ser  Ser  Pro  His  Val  Leu  Arg  Asn  Arg  Ala  Gln  Ser  Gly  Ser  Val  1,746
GGG  ATG  AGT  AGC  TCC  CCA  CAT  GTT  CTA  AGA  AAC  AGG  GCT  CAG  AGT  CGC  ACT  GTC

Pro  Gln  Phe  Lys  Lys  Val  Val  Phe  Gln  Glu  Phe  Thr  Asp  Gly  Ser  Phe  Thr  Gln  1,764
CCT  CAG  TTC  AAG  AAA  GTT  GTT  TTC  CAG  GAA  TTT  ACT  GAT  GGC  TCC  TTT  ACT  CAG

Pro  Leu  Tyr  Arg  Gly  Glu  Leu  Asn  Glu  His  Leu  Gly  Leu  Leu  Gly  Pro  Tyr  Ile  1,782
CCC  TTA  TAC  CGT  GGA  GAA  CTA  AAT  GAA  CAT  TTG  GGA  CTC  CTG  GGC  CCA  TAT  ATA

Arg  Ala  Glu  Val  Glu  Asp  Asn  Ile  MET  Val  Thr  Phe  Arg  Asn  Gln  Ala  Ser  Arg  1,800
AGA  GCA  GAA  GTT  GAA  GAT  AAT  ATC  ATG  CTA  ACT  TTC  AGA  AAT  CAG  GCC  TCT  CGT

Pro  Tyr  Ser  Phe  Tyr  Ser  Ser  Leu  Ile  Ser  Tyr  Glu  Glu  Asp  Gln  Arg  Gln  Gly  1,818
CCC  TAT  TCC  TTC  TAT  TCT  AGC  CTT  ATT  TCT  TAT  GAG  GAA  GAT  CAG  AGG  CAA  GGA

Ala  Glu  Pro  Arg  Lys  Asn  Phe  Val  Lys  Pro  Asn  Glu  Thr  Lys  Thr  Tyr  Phe  Trp  1,836
GCA  GAA  CCT  AGA  AAA  AAC  TTT  GTC  AAG  CCT  AAT  GAA  ACC  AAA  ACT  TAC  TTT  TGG

Lys  Val  Gln  His  His  MET  Ala  Pro  Thr  Lys  Asp  Glu  Phe  Asp  Cys  Lys  Ala  Trp  1,854
AAA  GTG  CAA  CAT  CAT  ATG  GCA  CCC  ACT  AAA  GAT  GAG  TTT  GAC  TGC  AAA  GCC  TGG

Ala  Tyr  Phe  Ser  Asp  Val  Asp  Leu  Glu  Lys  Asp  Val  His  Ser  Gly  Leu  Ile  Gly  1,872
GCT  TAT  TTC  TCT  GAT  GTT  GAC  CTG  GAA  AAA  GAT  GTG  CAC  TCA  GGC  CTG  ATT  GGA

Pro  Leu  Leu  Val  Cys  His  Thr  Asn  Thr  Leu  Asn  Pro  Ala  His  Gly  Arg  Gln  Val  1,890
CCC  CTT  CTG  CTC  TGC  CAC  ACT  AAC  ACA  CTG  AAC  CCT  GCT  CAT  CGG  AGA  CAA  GTG

Thr  Val  Gln  Glu  Phe  Ala  Leu  Phe  Phe  Thr  Ile  Phe  Asp  Glu  Thr  Lys  Ser  Trp  1,908
ACA  GTA  CAG  GAA  TTT  GCT  CTG  TTT  TTC  ACC  ATC  TTT  GAT  GAG  ACC  AAA  AGC  TGG

Thy  Phe  Thr  Glu  Asn  MET  Glu  Arg  Asn  Cys  Arg  Ala  Pro  Cys  Asn  Ile  Gln  MET  1,926
TAC  TTC  ACT  GAA  AAT  ATG  GAA  AGA  AAC  TGC  AGG  GCT  CCC  TGC  AAT  ATC  CAG  ATG

Glu  Asp  Pro  Thr  Phe  Lys  Glu  Asn  Thr  Arg  Phe  His  Ala  Ile  Asn  Gly  Tyr  Ile  1,944
GAA  GAT  CCC  ACT  TTT  AAA  GAG  AAT  TAT  CGC  TTC  CAT  GCA  ATC  AAT  GGC  TAC  ATA

MET  Asp  Thr  Leu  Pro  Gly  Leu  Val  MET  Ala  Gln  Asp  Gln  Arg  Ile  Arg  Trp  Tyr  1,962
ATG  GAT  ACA  CTA  CCT  GGC  TTA  GTA  ATG  GCT  CAG  GAT  CAA  AGG  ATT  CGA  TGG  TAT

Leu  Leu  Ser  MET  Gly  Ser  Asn  Glu  Asn  Ile  His  Ser  Ile  His  Phe  Ser  Gly  His  1,980
CTG  CTC  AGC  ATG  GGC  AGC  AAT  GAA  AAC  ATC  CAT  TCT  ATT  CAT  TTC  AGT  GGA  CAT

Val  Phe  Thr  Val  Arg  Lys  Lys  Glu  Glu  Tyr  Lys  MET  Ala  Leu  Tyr  Asn  Leu  Tyr  1,998
GTG  TTC  ACT  GTA  CGA  AAA  AAA  CAG  GAG  TAT  AAA  ATG  GCA  CTG  TAC  AAT  CTC  TAT

Pro  Gly  Val  Phe  Glu  Thr  Val  Glu  MET  Leu  Pro  Ser  Lys  Ala  Gly  Ile  Trp  Arg  2,016
CCA  GGT  CTT  TTT  GAG  ACA  GTG  GAA  ATG  TTA  CCA  TCC  AAA  GCT  GGA  ATT  TGG  CGG
```

```
Val  Glu  Cys  Leu  Ile  Gly  Glu  His  Leu  His  Ala  Gly  MET  Ser  Thr  Leu  Phe  Leu  2,034
GTG  GAA  TGC  CTT  ATT  GCC  GAG  CAT  CTA  CAT  GCT  GGG  ATG  AGC  ACA  CTT  TTT  CTG

Val  Tyr  Ser  Asn  Lys  Cys  Gln  Thr  Pro  Leu  Gly  MET  Ala  Ser  Gly  His  Ile  Arg  2,052
GTG  TAC  AGC  AAT  AAG  TGT  CAG  ACT  CCC  CTG  GGA  ATG  GCT  TCT  GGA  CAC  ATT  AGA

Asp  Phe  Gln  Ile  Thr  Ala  Ser  Gly  Gln  Tyr  Gly  Gln  Trp  Ala  Pro  Lys  Leu  Ala  2,070
GAT  TTT  CAG  ATT  ACA  GCT  TCA  GGA  CAA  TAT  GGA  CAG  TGG  GCC  CCA  AAG  CTG  GCC

Arg  Leu  His  Tyr  Ser  Gly  Ser  Ile  Asn  Ala  Trp  Ser  Thr  Lys  Glu  Pro  Phe  Ser  2,088
AGA  CTT  CAT  TAT  TCC  GGA  TCA  ATC  AAT  GCC  TGG  AGC  ACC  AAG  GAG  CCC  TTT  TCT

Trp  Ile  Lys  Val  Asp  Leu  Leu  Ala  Pro  MET  Ile  Ile  His  Gly  Ile  Lys  Thr  Gln  2,106
TGG  ATC  AAG  GTG  GAT  CTG  TTG  GCA  CCA  ATG  ATT  ATT  CAC  GGC  ATC  AAG  ACC  CAG

Gly  Ala  Arg  Gln  Lys  Phe  Ser  Ser  Leu  Tyr  Ile  Ser  Gln  Phe  Ile  Ile  MET  Tyr  2,124
GGT  GCC  CGT  CAG  AAG  TTC  TCC  AGC  CTC  TAC  ATC  TCT  CAG  TTT  ATC  ATC  ATG  TAT

Ser  Leu  Asp  Gly  Lys  Lys  Trp  Gln  Thr  Tyr  Arg  Gly  Asn  Ser  Thr  Gly  Thr  Leu  2,142
AGT  CTT  GAT  GGG  AAG  AAG  TGG  CAG  ACT  TAT  CGA  GGA  AAT  TCC  ACT  GGA  ACC  TTA

MET  Val  Phe  Phe  Gly  Asn  Val  Asp  Ser  Ser  Gly  Ile  Lys  His  Asn  Ile  Phe  Asn  2,160
ATG  GTC  TTC  TTT  GGC  AAT  GTG  GAT  TCA  TCT  GGG  ATA  AAA  CAC  AAT  ATT  TTT  AAC

Pro  Pro  Ile  Ile  Ala  Arg  Tyr  Ile  Arg  Leu  His  Pro  Thr  His  Tyr  Ser  Ile  Arg  2,178
CCT  CCA  ATT  ATT  GCT  CGA  TAC  ATC  CGT  TTG  CAC  CCA  ACT  CAT  TAT  AGC  ATT  CGC

Ser  Thr  Leu  Arg  MET  Glu  Leu  MET  Gly  Cys  Asp  Leu  Asn  Ser  Cys  Ser  MET  Pro  2,196
AGC  ACT  CTT  CGC  ATG  GAG  TTG  ATG  GGC  TGT  GAT  TTA  AAT  AGT  TGC  AGC  ATG  CCA

Leu  Gly  MET  Glu  Ser  Lys  Ala  Ile  Ser  Asp  Ala  Gln  Ile  Thr  Ala  Ser  Ser  Tyr  2,214
TTG  GGA  ATG  GAG  AGT  AAA  GCA  ATA  TCA  GAT  GCA  CAG  ATT  ACT  GCT  TCA  TCC  TAC

Phe  Thr  Asn  MET  Phe  Ala  Thr  Trp  Ser  Pro  Ser  Lys  Ala  Arg  Leu  His  Leu  Gln  2,232
TTT  ACC  AAT  ATG  TTT  GCC  ACC  TGG  TCT  CCT  TCA  AAA  GCT  CGA  CTT  CAC  CTC  CAA

Gly  Arg  Ser  Asn  Ala  Trp  Arg  Pro  Gln  Val  Asn  Asn  Pro  Lys  Glu  Trp  Leu  Gln  2,250
GGG  AGG  AGT  AAT  GCC  TGG  AGA  CCT  CAG  GTG  AAT  AAT  CCA  AAA  GAG  TGG  CTG  CAA

Val  Asp  Phe  Gln  Lys  Thr  MET  Lys  Val  Thr  Gly  Val  Thr  Thr  Gln  Gly  Val  Lys  2,268
GTG  GAC  TTC  CAG  AAG  ACA  ATG  AAA  GTC  ACA  GGA  GTA  ACT  ACT  CAG  GGA  GTA  AAA

Ser  Leu  Leu  Thr  Ser  MET  Tyr  Val  Lys  Glu  Phe  Leu  Ile  Ser  Ser  Ser  Gln  Asp  2,286
TCT  CTG  CTT  ACC  ACC  ATG  TAT  GTG  AAG  GAG  TTC  CTC  ATC  TCC  ACC  AGT  CAA  GAT

Gly  His  Gln  Trp  Thr  Leu  Phe  Phe  Gln  Asn  Gly  Lys  Val  Lys  Val  Phe  Gln  Gly  2,304
GGC  CAT  CAG  TGG  ACT  CTC  TTT  TTT  CAG  AAT  GGC  AAA  CTA  AAG  GTT  TTT  CAG  GGA

Asn  Gln  Asp  Ser  Phe  Thr  Pro  Val  Val  Asn  Ser  Leu  Asp  Pro  Pro  Leu  Leu  Thr  2,322
AAT  CAA  GAC  TCC  TTC  ACA  CCT  GTG  GTG  AAC  TCT  CTA  GAC  CCA  CCG  TTA  CTG  ACT

Arg  Tyr  Leu  Arg  Ile  His  Pro  Gln  Ser  Trp  Val  His  Gln  Ile  Ala  Leu  Arg  MET  2,340
CGC  TAC  CTT  CGA  ATT  CAC  CCC  CAG  AGT  TCG  GTG  CAC  CAG  ATT  GCC  CTG  AGG  ATG
```

14/14                                                                8

```
Glu  Val  Leu  Gly  Cys  Glu  Ala  Gln  Asp  Leu  Tyr  End                        2,352
GAG  GTT  CTG  GGC  TGC  GAG  GCA  CAG  GAC  CTC  TAC  TGA  GGGTGGCCACTGCATCCCACCTGCCAGTG
```

CCGTCACCTCTCCCTCCTCAGCTCCAGGGCATGTGTCCCTCCCTGGCTTGCTTCTACCTTTGTGCTAAATCCTAGCAGACACTCCCTTG

AAGCCTCCTGAATTAACTATCATCAGTCCTGCATTTCTTTGGTGGGGGGCCAGGAGGGTGCATCCATTTTAACTTAACTCTTACCTATT

TTCTGCAGCTGCTCCCAGA